(19) Europäisches Patentamt
European Patent Office
Office européen des brevets

(11) EP 1 103 554 B1

(12) # FASCICULE DE BREVET EUROPEEN

(45) Date de publication et mention de la délivrance du brevet: **12.03.2003 Bulletin 2003/11**

(21) Numéro de dépôt: **00403255.3**

(22) Date de dépôt: **22.11.2000**

(51) Int Cl.[7]: **C07D 491/04**, C07D 491/14, A61K 31/47, A61P 35/00, C07D 221/18, C07D 221/16, C07D 491/16, C07D 495/16, C07D 495/04
// (C07D491/04, 307:00, 221:00), (C07D491/14, 317:00, 307:00, 221:00), (C07D491/16, 311:00, 307:00, 221:00), (C07D495/04, 333:00, 221:00)

(54) **Dérivés de dihydrofuro-(3,4-b) quinoléin-1-ones, leur procédé de préparation et les compositions pharmaceutiques qui les contiennent**

Dihydrofuro-(3,4-b)-chinolein-1-on Derivate, Verfahren zu ihrer Herstellung und sie enthaltende pharmazeutische Zusammensetzungen

Derivatives of dihydrofuro-(3,4-b)-quinolein-1-ones, process of préparation and phamaceutical compositions containing them

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **24.11.1999 FR 9914771**

(43) Date de publication de la demande:
**30.05.2001 Bulletin 2001/22**

(73) Titulaire: **LES LABORATOIRES SERVIER**
**92200 Neuilly sur Seine (FR)**

(72) Inventeurs:
- **Husson, Henri-Philippe**
  **78460 Chevreuse (FR)**
- **Giorgi-Renault, Sylviane**
  **75005 Paris (FR)**
- **Tratrat, Christophe**
  **75019 Paris (FR)**
- **Atassi, Ghanem**
  **92210 Saint-Cloud (FR)**
- **Pierre, Alain**
  **78580 Les Alluets Le Roi (FR)**
- **Renard, Pierre**
  **78150 Le Chesnay (FR)**
- **Pfeiffer, Bruno**
  **95320 Saint Leu La Foret (FR)**

(56) Documents cités:
**EP-A- 0 382 687**
**EP-A- 0 585 913**
**EP-A- 0 634 169**
**WO-A-99/08528**
**FR-A- 2 219 786**

- **Y. HITOTSUYANAGI ET AL.: TETRAHEDRON LETTERS, vol. 40, no. 51, 1999, pages 9107-9110, XP004184816 OXFORD GB**
- **HITOTSUYANAGI, YUKIO ET AL: "4-Aza-2,3-dehydro-4-deoxypodophyllotoxins : simple Aza-podophyllotoxin analogues possessing potent cytotoxicity" BIOORG. MED. CHEM. LETT. (2000), 10(4), 315-317 , XP004189922**
- **DATABASE CHEMABS [en ligne] CHEMICAL ABSTRACTS SERVICE, COLUMBUS, OHIO, US; MATSUO, KEIZO ET AL: "Synthesis and reaction of 9-aryl-1H,3H-thieno[3,4-b]quinolin-1-ones" retrieved from STN Database accession no. 119:225860 XP002156855 & CHEM. EXPRESS (1993), 8(6), 389-92 ,**
- **Y. HITOTSUYANAGI ET AL.: BIOORG. MED. CHEM. LETT., vol. 5, no. 10, 1995, pages 1039-1042, XP004135507**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 103 554 B1

• **ITO A ET AL: "ANTIMUTAGENIC CONSTITUENTS OF CASIMIROA EDULIS WITH POTENTIAL CANCER CHEMOPREVENTIVE ACTIVITY" JOURNAL OF AGRICULTURAL AND FOOD CHEMISTRY,US,AMERICAN CHEMICAL SOCIETY. WASHINGTON, vol. 46, no. 9, 1998, pages 3509-3516, XP000872160 ISSN: 0021-8561**

Remarques:

Le dossier contient des informations techniques présentées postérieurement au dépôt de la demande et ne figurant pas dans le présent fascicule.

## Description

La présente invention concerne de nouveaux dérivés de dihydrofuro-[3,4-b]quinoléin-1-ones, leur procédé de préparation, les compositions pharmaceutiques qui les contiennent ainsi que leur utilisation en tant qu'anti-cancéreux.

Les besoins de la thérapeutique anticancéreuse exigent le développement constant de nouveaux agents antitumoraux, dans le but d'obtenir des médicaments à la fois plus actifs et mieux tolérés.

Les composés de l'invention, outre le fait qu'ils soient nouveaux, présentent des propriétés antitumorales très intéressantes.

Des composés de structure proche ont déjà été décrits dans la littérature, notamment des dérivés de furo-[3,4-b]-quinoléin-1-ones en tant qu'anti-ostéoporotiques (brevet EP 0 634 169), de thiéno [3,4-b] quinoléin-1-ones (Chem. Express 1993, 8 (6), 389-92), et de 3,4,9,10-tétrahydro-1 (2*H*)-acridinones, en tant qu'antihypertenseurs (brevet FR 2219786).

En revanche, aucune activité cytotoxique n'a jamais été décrite pour ces dérivés.

D'autre part, des analogues de diazapodophyllotoxines ont été décrites pour leur activité cytotoxique dans Bioorg. Med. Chem. Lett. 1995, 5 (10), pp. 1039-1042.

Plus spécifiquement, la présente invention concerne les composés de formule (I) :

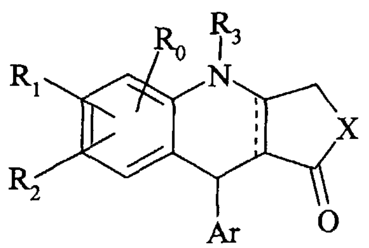

(I)

dans laquelle :

➢ ---- représente une liaison double,

➢ $R_0$ représente un atome d'hydrogène ou un groupement hydroxy ou alkoxy ($C_1$-$C_6$) linéaire ou ramifié,

➢ $R_1$ et $R_2$, identiques ou différents, représentent chacun :

- un atome d'hydrogène,
- un atome d'halogène,
- un groupement alkyle ($C_1$-$C_6$) linéaire ou ramifié,
- un groupement alkoxy ($C_1$-$C_6$) linéaire ou ramifié,
- un groupement hydroxy,
- un groupement polyhalogénoalkyle ($C_1$-$C_6$) linéaire ou ramifié,
- un groupement nitro,
- un groupement amino éventuellement substitué par un ou deux groupements alkyle ($C_1$-$C_6$) linéaire ou ramifié,
- un groupement de formule

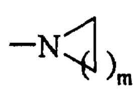

dans laquelle m représente un entier tel que 1 ≤ m ≤ 4,

ou forment ensemble avec les atomes de carbone qui les portent un groupement mono-ou bicyclique de 5 à 12 chaînons, aromatique ou non-aromatique, contenant éventuellement 1 ou 2 hétéroatomes choisis parmi O, S et N,

➢ $R_3$ représente un atome d'hydrogène ou un groupement de formule $R_4$ dans laquelle $R_4$ représente :

- un groupement aryle,
- un groupement hétéroaryle,
- un groupement cycloalkyle ($C_3$-$C_8$),
- un groupement alkyle ($C_1$-$C_6$) linéaire ou ramifié éventuellement substitué par un groupement aryle, par un groupement hétéroaryle, par un groupement hydroxy, par un groupement alkoxy ($C_1$-$C_6$) linéaire ou ramifié, ou par un groupement de formule $NR_5R_6$ dans laquelle $R_5$ et $R_6$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté,
- ou un groupement de formule $COR_7$, dans laquelle $R_7$ représente un groupement :
  - aryle,
  - alkyle ($C_1$-$C_6$) linéaire ou ramifié (éventuellement substitué par un groupement de formule $NR_8R_9$ dans laquelle $R_8$ et $R_9$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté),
  - amino éventuellement substitué par un ou plusieurs groupements aryle, hétéroaryle, ou alkyle ($C_1$-$C_6$) linéaire ou ramifié éventuellement substitué par un groupement de formule $NR_8R_9$ dans laquelle $R_8$ et $R_9$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté,
  - ou $OR_{10}$ dans laquelle $R_{10}$ représente un atome d'hydrogène ou un groupement aryle, ou alkyle ($C_1$-$C_6$) linéaire ou ramifié éventuellement substitué par un groupement de formule $NR_8R_9$ dans laquelle $R_8$ et $R_9$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté,

➢ X représente un atome d'oxygène, de soufre ou un groupement $-CH_2-$ ou $-CH_2-CH_2-$,

➢ Ar représente un groupement aryle, hétéroaryle ou arylalkyle ($C_1$-$C_6$) linéaire ou ramifié,

leurs isomères optiques, leurs sels d'addition à un acide pharmaceutiquement acceptable ainsi que leurs hydrates et leurs solvates,
à l'exclusion de la 7-chloro-9-phényl-4,9-dihydrothiéno[3,4-b]quinoléin-1(3*H*)-one, de la 7-chloro-4-méthyl-9-phényl-4,9dihydrothiéno[3,4-b]quinoléin-1(3*H*)-one, de la 7-chloro-4-[2-(diéthylamino)-éthyl]-9phényl-4,9-dihydrothiéno [3,4-b]quinoléin-1(3*H*)-one, de la 9-phényl-3,4,9,10-tétrahydro-1(2*H*)-acridinone, de la 9-(2-thiényl)-3,4,9,10-tétrahydro-1(2*H*)-acridinone et de la 9-(4-méthylphényl)-3,4,9,10-tétrahydro-1 (2*H*)-acridinone.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique, camphorique.

Par groupement aryle, on entend phényle, biphényle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle ($C_1$-$C_6$) linéaire ou ramifié, hydroxy, alkoxy ($C_1$-$C_6$) linéaire ou ramifié, polyhalogénoalkyle ($C_1$-$C_6$) linéaire ou ramifié, amino (substitué éventuellement par un ou plusieurs groupements alkyle ($C_1$-$C_6$) linéaire ou ramifié), nitro, acyle ($C_1$-$C_6$) linéaire ou ramifié ou alkylènedioxy ($C_1$-$C_2$).

Par groupement hétéroaryle, on entend un groupement aromatique mono- ou bicyclique de 5 à 12 chaînons contenant un, deux ou trois hétéroatomes choisis parmi oxygène, azote ou soufre étant entendu que l'hétéroaryle peut être éventuellement substitué par un ou plusieurs atomes ou groupements, identiques ou différents, choisis parmi les atomes d'halogène et les groupements alkyle ($C_1$-$C_6$) linéaire ou ramifié, hydroxy, alkoxy ($C_1$-$C_6$) linéaire ou ramifié, polyhalogénoalkyle ($C_1$-$C_6$) linéaire ou ramifié, ou amino (substitué éventuellement par un ou plusieurs groupements alkyle ($C_1$-$C_6$) linéaire ou ramifié). Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, quinolyle, isoquinolyle, pyrimidinyle.

Par hétérocycle azoté, on entend un groupement monocyclique saturé de 5 à 7 chaînons contenant un, deux

ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre. Les hétérocycles azotés préférés sont les groupements pyrrolidinyle, pipéridyle, morpholinyle ou pipérazinyle.

Parmi les groupements mono- ou bicycliques de 5 à 12 chaînons, aromatiques ou non-aromatiques, contenant éventuellement 1 ou 2 hétéroatomes choisis parmi O, S et N, on peut citer à titre non limitatif les groupements phénylène, naphtylène, cyclopenténylène, et les groupements de formules $G_1$ à $G_5$ :

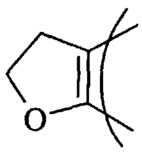

$G_1$

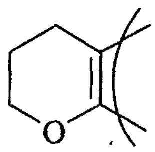

$G_2$

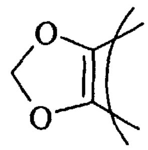

$G_3$

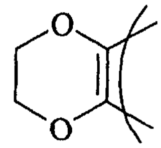

$G_4$

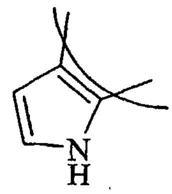

$G_5$

Les composés préférés de formule (I) sont ceux pour lesquels $R_0$ représente un atome d'hydrogène.

Un aspect avantageux de l'invention concerne les composés de formule (I) pour lesquels $R_1$ et $R_2$, identiques ou différents, représentent chacun :

- un atome d'hydrogène,
- un atome d'halogène,
- un groupement alkyle ($C_1$-$C_6$) linéaire ou ramifié,
- un groupement alkoxy ($C_1$-$C_6$) linéaire ou ramifié,
- un groupement hydroxy,
- un groupement polyhalogénoalkyle ($C_1$-$C_6$) linéaire ou ramifié,
- un groupement nitro,
- un groupement amino éventuellement substitué par un ou deux groupements alkyle ($C_1$-$C_6$) linéaire ou ramifié, ou
- un groupement de formule

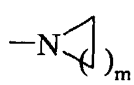

dans laquelle m représente un entier tel que $1 \leq m \leq 4$.

Un autre aspect avantageux de l'invention concerne les composés de formule (I) pour lesquels $R_1$ et $R_2$ forment ensemble avec les atomes de carbone qui les portent un groupement mono- ou bicyclique de 5 à 12 chaînons, aromatique ou non-aromatique, contenant éventuellement 1 ou 2 hétéroatomes choisis parmi O, S et N.
Parmi ceux-ci, on préférera tout particulièrement ceux pour lesquels $R_1$ et $R_2$ forment ensemble, avec les atomes de carbone qui les portent, un groupement phénylène ou un groupement de formule $G_3$ ou $G_4$ tels que définis précédemment.

Un autre aspect avantageux de l'invention concerne les composés de formule (I) pour lesquels X représente un atome d'oxygène ou de soufre.

Un autre aspect avantageux de l'invention concerne les composés de formule (I) pour lesquels X représente un groupement $-CH_2-$ ou $-CH_2-CH_2-$.

Un autre aspect avantageux de l'invention concerne les composés de formule (I) pour lesquels Ar représente un groupement aryle. Parmi ceux-ci, on préférera tout particulièrement ceux pour lesquels Ar représente un groupement phényle éventuellement substitué.

Un autre aspect avantageux de l'invention concerne les composés de formule (I) pour lesquels Ar représente un groupement hétéroaryle.

Parmi les composés préférés de l'invention, on peut citer plus particulièrement :

- la (±) 4-méthyl-6,7-méthylènedioxy-9-(3,4,5-triméthoxyphényl)-4,9-dihydrofuro[3,4-b] quinoléin-1(3*H*)-one, ainsi que ses isomères optiques, ses hydrates et ses solvates,

- la (±) 6,7-méthylènedioxy-9-(3,4,5-triméthoxyphényl)-4,9-dihydrofuro[3,4-b]quinoléin-1(3*H*)-one, ses isomères optiques, ses hydrates, ses solvates ainsi que ses sels d'addition à un acide pharmaceutiquement acceptable,
- la (±) 6-méthoxy-9-(3,4,5-triméthoxyphényl)-4,9-dihydrofuro[3,4-b]quinoléin-1(3*H*)-one, ses isomères optiques, ses hydrates, ses solvates ainsi que ses sels d'addition à un acide pharmaceutiquement acceptable,
- la (±) 7-(3,4,5-triméthoxyphényl)7,11-dihydrobenzo[h]furo[3,4-b]quinoléin-8(10*H*)-one, ses isomères optiques, ses hydrates, ses solvates ainsi que ses sels d'addition à un acide pharmaceutiquement acceptable,
- la (±) 6,7-méthylènedioxy-9-(3,4,5-triméthoxyphényl)-2,3,4,9-tétrahydrocyclopenta[b] quinoléin-1-one, ses isomères optiques, ses hydrates, ses solvates ainsi que ses sels d'addition à un acide pharmaceutiquement acceptable,
- la (±) 6,7-éthylènedioxy-9-(3,4,5-triméthoxyphényl)-4,9-dihydrofuro[3,4-b]quinoléin-1(3*H*)-one, ses isomères optiques, ses hydrates, ses solvates ainsi que ses sels d'addition à un acide pharmaceutiquement acceptable,
- et la (±) 7-(3,4,5-triméthoxyphényl)-7,9,10,11-tétrahydro-8*H*-benzo[h]cyclopenta[b] quinoléin-8-one, ses isomères optiques, ses hydrates, ses solvates ainsi que ses sels d'addition à un acide pharmaceutiquement acceptable.

L'invention s'étend également à un procédé de préparation des composés de formule (I) caractérisé en ce que l'on met en réaction un composé de formule (II):

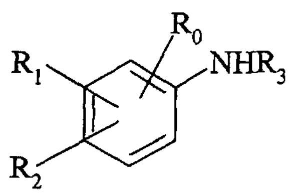

(II)

dans laquelle $R_0$, $R_1$, $R_2$ et $R_3$ ont la même signification que dans la formule (I),
avec un composé de formule (III) :

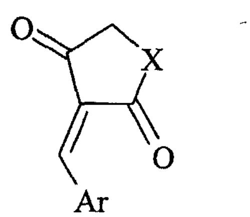

(III)

dans laquelle X et Ar ont la même signification que dans la formule (I),
pour conduire au composé de formule (I),
que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

- Lorsque $R_3$ ne représente pas un atome d'hydrogène, le composé de formule (II) peut être obtenu par réaction du composé de formule (IV):

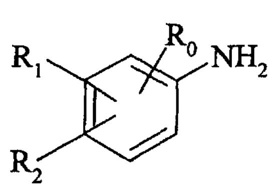

(IV)

dans laquelle $R_0$, $R_1$, et $R_2$, ont la même significaiton que précédemment,
avec un composé de formule (V) :

$R_4$-Y (V)

dans laquelle $R_4$ a la même signification que dans la formule (I), et Y représente un groupement partant tel que, par exemple, un atome d'halogène ou un groupement mésylate, tosylate ou trifluorométhanesulfonate.

- Lorsque $R_3$ représente un groupement de formule $-CH_2R'_3$ dans laquelle $R'_3$ représente un groupement aryle, un groupement hétéroaryle ou un groupement alkyle ($C_1$-$C_5$) linéaire ou ramifié éventuellement substitué par un groupement aryle, par un groupement hétéroaryle, par un groupement hydroxy, par un groupement alkoxy ($C_1$-$C_6$) linéaire ou ramifié éventuellement substitué, ou par un groupement de formule $NR_5R_6$ dans laquelle $R_5$ et $R_6$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté, le composé de formule (II) peut également être obtenu par réaction en présence d'un agent réducteur du composé de formule (IV) avec un composé de formule (VI):

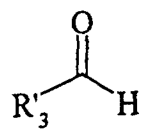

(VI)

dans laquelle $R'_3$ a la même signification que précédemment.

- Lorsque $R_3$ représente un groupement de formule $COR'_7$ dans laquelle $R'_7$ représente un groupement aryle, le composé de formule (II) peut également être obtenu par réaction du composé de formule (VII) :

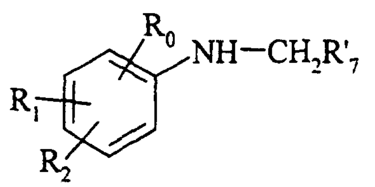

(VII)

dans laquelle $R_0$, $R_1$, $R_2$ et $R'_7$ ont la même signification que précédemment, avec un agent oxydant.

- Lorsque $R_3$ représente un groupement $CH_3$, le composé de formule (II) peut également être obtenu par réaction du composé de formule (IV) avec l'acide formique en présence d'un agent réducteur.

- Le composé de formule (III) est obtenu par réaction du composé de formule (VIII):

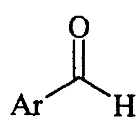

(VIII)

dans laquelle Ar a la même signification que précédemment, avec un composé de formule (IX):

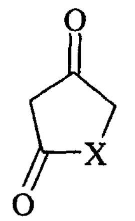

(IX)

dans laquelle X a la même signification que précédemment, selon le procédé décrit dans J. Org. Chem. 1978, 43, 1541.

L'invention s'étend également à un deuxième procédé de préparation des composés de formule (I), caractérisé en ce que l'on met en réaction dans le même pot un composé de formule (II), un composé de formule (VIII) et un composé de formule (IX),
pour conduire au composé de formule (I),
que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

Les composés de la présente invention, outre le fait qu'ils soient nouveaux, présentent des propriétés pharmacologiques intéressantes. Ils ont des propriétés cytotoxiques qui les rendent utiles dans le traitement des cancers.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse, intramusculaire ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention et ne la limitent en aucune façon.

Les produits de départ utilisés sont des produits connus ou préparés selon des modes préparatoires connus.

Les préparations A à S conduisent à des intermédiaires de synthèse, utiles pour la préparation des composés de l'invention.

Les structures des composés décrits dans les exemples ont été déterminées selon les techniques spectrométriques usuelles (infrarouge, RMN, spectrométrie de masse).

## PREPARATION A : 3-(3,4,5-Triméthoxybenzylidène)-2,4-(3*H*,5*H*)-furanedione

Le produit attendu est obtenu selon le procédé décrit dans J. Org. Chem. 1978, 43, 1541, à partir de 3,4,5-triméthoxybenzaldéhyde et d'acide tétronique.

## PREPARATION B : 3-(4-Hydroxy-3,5-diméthoxybenzylidène)-2,4-(3*H*,5*H*)-furanedione

Le produit attendu est obtenu selon le procédé décrit dans J. Org. Chem. 1978, 43, 1541, à partir de 4-hydroxy-3,5-diméthoxybenzaldéhyde et d'acide tétronique.

## PREPARATION C : 3-(3-Pyridyl-méthylène)-2,4-(3*H*,5*H*)-furanedione

Le produit attendu est obtenu selon le procédé décrit dans J. Org. Chem. 1978, 43, 1541, à partir de nicotinaldéhyde et d'acide tétronique.

## PREPARATION D : 3-(3-Méthoxy-4,5-méthylènedioxybenzylidène)-2,4-(3*H*,5*H*)-furanedione

Le produit attendu est obtenu selon le procédé décrit dans J. Org. Chem. 1978, 43, 1541, à partir de 3-méthoxy-4,5-méthylènedioxybenzaldéhyde et d'acide tétronique.

**PREPARATION E : 3-(2,3,4-Triméthoxybenzylidène)-2,4-(3*H*,5*H*)-furanedione**

Le produit attendu est obtenu selon le procédé décrit dans J. Org. Chem. 1978, 43, 1541, à partir de 2.3,4-triméthoxybenzaldéhyde et d'acide tétronique.

**PREPARATION F : 3-(3,4-Diméthoxybenzylidène)-2,4-(3*H*,5*H*)-furanedione**

Le produit attendu est obtenu selon le procédé décrit dans J. Org. Chem. 1978, 43, 1541, à partir de 3,4-diméthoxybenzaldéhyde et d'acide tétronique.

**PREPARATION G : 3-(3-Hydroxy-4-méthoxybenzylidène)-2,4-(3*H*,5*H*)-furanedione**

Le produit attendu est obtenu selon le procédé décrit dans J. Org. Chem. 1978, 43, 1541, à partir de 3-hydroxy-4-méthoxybenzaldéhyde et d'acide tétronique.

**PREPARATION H : 3-(4-Méthoxybenzylidène)-2,4-(3*H*,5*H*)-furanedione**

Le produit attendu est obtenu selon le procédé décrit dans J. Org. Chem. 1978, 43, 1541, à partir de 4-méthoxybenzaldéhyde et d'acide tétronique.

**PREPARATION I: 3-(3-Méthoxybenzylidène)-2,4-(3*H*,5*H*)-furanedione**

Le produit attendu est obtenu selon le procédé décrit dans J. Org. Chem. 1978, 43, 1541, à partir de 3-méthoxybenzaldéhyde et d'acide tétronique.

**PREPARATION J : 3-Benzylidène-2,4-(3*H*,5*H*)-furanedione**

Le produit attendu est obtenu selon le procédé décrit dans J. Org. Chem. 1978, 43, 1541, à partir de benzaldéhyde et d'acide tétronique.

**PREPARATION K : 3-(2-Furyl-méthylène)-2,4-(3*H*,5*H*)-furanedione**

Le produit attendu est obtenu selon le procédé décrit dans J. Org. Chem. 1978, 43, 1541, à partir de 2-furaldéhyde et d'acide tétronique.

**PREPARATION L : 3-(3,4,5-Triméthoxybenzylidène)-1,3-cyclopentanedione**

Le produit attendu est obtenu selon le procédé décrit dans J. Org. Chem. 1978, 43, 1541, à partir de 3,4,5-triméthoxybenzaldéhyde et de 1,3-cyclopentanedione.

**PREPARATION M : 3-(4,5-Méthylènedioxybenzylidène)-2,4-(3*H*,5*H*)-furanedione**

Le produit attendu est obtenu selon le procédé décrit dans J. Org. Chem. 1978, 43, 1541, à partir de 4,5-méthylènedioxybenzaldéhyde et d'acide tétronique.

**PREPARATION N : 3-(4-Chlorobenzylidène)-2,4-(3*H*,5*H*)-furanedione**

Le produit attendu est obtenu selon le procédé décrit dans J. Org. Chem. 1978, 43, 1541, à partir de 4-chlorobenzaldéhyde et d'acide tétronique.

**PREPARATION O : 3-(4-Nitrobenzylidène)-2,4-(3*H*,5*H*)-furanedione**

Le produit attendu est obtenu selon le procédé décrit dans J. Org. Chem. 1978, 43, 1541, à partir de 4-nitrobenzaldéhyde et d'acide tétronique.

**PREPARATION P : 3-(3-Nitrobenzylidène)-2,4-(3*H*,5*H*)-furanedione**

Le produit attendu est obtenu selon le procédé décrit dans J. Org. Chem. 1978, 43, 1541, à partir de 3-nitrobenzaldéhyde et d'acide tétronique.

**PREPARATION Q : 3-(4-Diméthylaminobenzylidène)-2,4-(3*H*,5*H*)-furanedione**

Le produit attendu est obtenu selon le procédé décrit dans J. Org. Chem. 1978, 43, 1541, à partir de 4-diméthylaminobenzaldéhyde et d'acide tétronique.

**PREPARATION R : 3-(4-Pyridyl-méthylène)-2,4-(3*H*,5*H*)-furanedione**

Le produit attendu est obtenu selon le procédé décrit dans J. Org. Chem. 1978, 43, 1541, à partir d'isonicotinaldéhyde et d'acide tétronique.

**PREPARATION S : 3-(2-Pyridyl-méthylène)-2,4-(3*H*,5*H*)-furanedione**

Le produit attendu est obtenu selon le procédé décrit dans J. Org. Chem. 1978, 43, 1541, à partir de 2-pyridinecarboxaldéhyde et d'acide tétronique.

**EXEMPLE 1 : (±) 4-Méthyl-6,7-méthylènedioxy-9-(3,4,5-triméthoxyphényl)-4,9-dihydrofuro[3,4-b]quinoléin-1(3*H*)-one**

*Stade A : N-méthyl-3,4-méthylènedioxyaniline*

Une solution de 3,4-méthylènedioxyaniline (10 mmoles) dans l'acide formique (36 ml) est chauffée à reflux pendant 1 heure. Après élimination de l'acide formique en excès, le résidu obtenu est dilué dans l'eau, puis extrait au dichlorométhane. Les phases organiques réunies sont évaporées. Le résidu obtenu est mis en solution dans l'éther, puis de l'hydrure d'aluminium et de lithium (42 mmoles) est ajouté à 10°C, par petites portions. Après 3 heures d'agitation à température ambiante, de l'eau et de la soude à 10 % sont ajoutées puis le mélange est filtré sur célite. Le filtrat est séché, évaporé et le résidu obtenu est purifié par chromatographie sur silice en utilisant le dichlorométhane comme éluant pour conduire au produit attendu sous forme d'une huile.

*Stade B : (±)4-Méthyl-6,7-méthylènedioxy-9-(3,4,5-triméthoxyphényl)-4,9-dihydrofuro[3,4-b]quinoléin-1(3*H*)-one*

Au composé obtenu dans le stade précédent (10 mmoles) en suspension dans l'éthanol sont ajoutées 10 mmoles du composé décrit dans la préparation A, puis le mélange est chauffé au reflux pendant 30 minutes. Après retour à température ambiante, le précipité obtenu est filtré, lavé puis recristallisé pour conduire au produit attendu.
*Point de fusion :* 226°C

| *Microanalyse élémentaire :* | | | |
|---|---|---|---|
| | *%C* | *%H* | *%N* |
| *Calculé* | *64,23* | *5,14* | *3,40* |
| *Trouvé* | *64,11* | *5,09* | *3,26* |

**EXEMPLE 2 : (±) 4-Méthyl-6,7-méthylènedioxy-9-(3,4,5-triméthoxy phényl)-3a,4,9,9a-tétrahydrofuro[3,4-b]quinoléin-1(3*H*)-one**

Une suspension du composé décrit dans l'Exemple 1 (10 mmoles) dans l'éthanol est placée sous pression d'hydrogène (50 psi) à température ambiante en présence de charbon palladié. Après filtration du catalyseur, le solvant est évaporé et le résidu obtenu est purifié par chromatographie sur silice en utilisant le dichlorométhane comme éluant.

**EXEMPLE 3 : (±) 6,7-Méthylènedioxy-4-(4-nitrobenzyl)-9-(3,4,5-triméthoxy phényl)-4,9-dihydrofuro[3,4-b]quinoléin-1(3*H*)-one**

*Stade A : N-(4-Nitrobenzyl)-3,4-méthylènedioxyaniline*

Une solution de 3,4-méthylènedioxyaniline (10 mmoles) et de 4-nitrobenzaldéhyde (10 mmoles) dans le méthanol est agitée à température ambiante pendant 18 heures. Le précipité obtenu est filtré, lavé puis mis en solution

dans le tétrahydrofurane. Après addition d'acide acétique et de borohydrure de sodium (25 mmoles), le mélange réactionnel est agité à température ambiante pendant 1 heure, puis le solvant est évaporé et le résidu brut est purifié par chromatographie sur silice en utilisant le dichlorométhane comme éluant.
*Point de fusion : 114°C*

*Stade B : (±) 6,7-Méthylènedioxy-4-(4-nitrobenzyl)-9-(3,4,5-triméthoxy phényl)-4,9-dihydrofuro[3,4-b]quinoléin-1(3H)-one*

Le produit attendu est obtenu selon le procédé décrit dans le stade B de l'Exemple 1 à partir du composé obtenu dans le stade précédent et du composé décrit dans la Préparation A.
*Point de fusion : 240°C*

| Microanalyse élémentaire : (hémihydrate) | | | |
|---|---|---|---|
| | % C | % H | % N |
| Calculé | 62,11 | 4,80 | 5,17 |
| Trouvé | 62,43 | 4,49 | 5,13 |

**EXEMPLE 4 : (±) 4-(4-Chlorobenzyl)-6,7-méthylènedioxy-9-(3,4,5-triméthoxy phényl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de 4-chloro-benzaldéhyde et du composé décrit dans la Préparation A.
*Point de fusion : > 260°C*

| Microanalyse élémentaire : (hémihydrate) | | | |
|---|---|---|---|
| | %C | %H | %N |
| Calculé | 63,34 | 4,74 | 2,64 |
| Trouvé | 63,51 | 4,73 | 2,59 |

**EXEMPLE 5 : (±) 4-(3-Hydroxybenzyl)-6,7-méthylènedioxy-9-(3,4,5-triméthoxyphényl)-4,9-dihydrofuro[3,4-b] quinoléin-1(3*H*)-one**

Le produit attendu est obtenu selon le procédé décrit dans l'Exemple 3 à partir de 3,4-méthylènedioxyaniline, de 3-hydroxybenzaldéhyde et du composé décrit dans la Préparation A.
*Point de fusion : 245°C*

| Microanalyse élémentaire : (hydrate) | | | |
|---|---|---|---|
| | %C | %H | %N |
| Calculé | 64,49 | 5,22 | 2,68 |
| Trouvé | 64,71 | 4,99 | 2,58 |

**EXEMPLE 6 : (±) 6,7-Méthylènedioxy-4-(3,4-méthylènedioxybenzyl)-9-(3,4,5-triméthoxyphényl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de 3,4-méthylènedioxybenzaldéhyde et du composé décrit dans la Préparation A.
*Point de fusion : > 260°C*

| Microanalyse élémentaire : (hydrate) | | | |
|---|---|---|---|
| | %C | %H | %N |
| Calculé | 63,38 | 4,95 | 2,55 |
| Trouvé | 63,72 | 4,73 | 2,51 |

**EXEMPLE 7 : (±) 6,7-Méthylènedioxy-4-(3,4,5-triméthoxybenzyl)-9-(3,4,5-triméthoxyphényl)-4,9-dihydrofuro [3,4-b]quinoléin-1(3*H*)-one**

Le produit attendu est obtenu selon le procédé décrit dans l'Exemple 3 à partir de 3,4-méthylènedioxyaniline, de 3,4,5-triméthoxybenzaldéhyde et du composé décrit dans la Préparation A.
*Point de fusion : 190°C*

| *Microanalyse élémentaire : (hémihydrate)* | | | |
|---|---|---|---|
| | *%C* | *%H* | *%N* |
| *Calculé* | *63,47* | *5,50* | *2,39* |
| *Trouvé* | *63,58* | *5.59* | *2,65* |

**EXEMPLE 8 : (±) 4-(3,5-Diméthoxy-4-hydroxybenzyl)-6,7-méthylènedioxy-9-(3,4,5-triméthoxyphényl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de 3,5-diméthoxy-4-hydroxy-benzaldéhyde et du composé décrit dans la Préparation A.
*Point de fusion : 242°C*

| *Microanalyse élémentaire :* | | | |
|---|---|---|---|
| | *%C* | *%H* | *%N* |
| *Calculé* | *63,94* | *5,19* | *2,49* |
| *Trouvé* | *63,72* | *5,21* | *2,46* |

**EXEMPLE 9 : (±) 4-(2-Hydroxyéthyl)-6,7-méthylènedioxy-9-(3,4,5-triméthoxyphényl)-4,9-dihydrofuro[3,4-b]quinoléin-1(3*H*)-one**

*Stade A : N-(3,4-Méthylènedioxyphényl)-2-aminoéthanol*

A 10 mmoles de 3,4-méthylènedioxyaniline en solution dans l'éthanol sont ajoutées 27 mmoles d'acétate de sodium et 15 mmoles de 2-bromoéthanol. Après 24 heures de chauffage au reflux, le mélange réactionnel est ramené à température ambiante, de l'eau est ajoutée puis la phase aqueuse est extraite au dichlorométhane. Les phases organiques sont rassemblées, le solvant est évaporé et le résidu brut est purifié par chromatographie sur silice en utilisant un mélange dichlorométhane/acétate d'éthyle 10/90 comme éluant, pour conduire au produit attendu sous la forme d'une huile.

*Stade B : (±) 4-(2-Hydroxyéthyl)-6,7-méthylènedioxy-9 -(3,4,5-triméthoxyphényl)-4,9-dihydrofuro[3,4-b]quinoléin-1 (3*H*)-one*

Le produit attendu est obtenu selon le procédé décrit dans le stade B de l'Exemple 1 à partir du composé décrit dans le stade précédent et du composé décrit dans la Préparation A.
*Point de fusion : > 260°C*

| *Microanalyse élémentaire : (hémihydrate)* | | | |
|---|---|---|---|
| | *%C* | *%H* | *%N* |
| *Calculé* | *61,33* | *5,37* | *3,11* |
| *Trouvé* | *61,54* | *5,28* | *2,94* |

**EXEMPLE 10 : (±) 6,7-Méthylènedioxy-9-(3,4,5-triméthoxyphényl)-4,9-dihydrofuro[3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation A.
*Point de fusion : > 260°C*

| *Microanalyse élémentaire : (hémihydrate)* | | | |
|---|---|---|---|
| | *%C* | *%H* | *%N* |
| *Calculé* | *63,47* | *4,82* | *3,52* |
| *Trouvé* | *63,32* | *4,86* | *3,45* |

**EXEMPLE 11 : (±) 6,7-Méthylènedioxy-4-propyl-9-(3,4,5-triméthoxyphényl)-4,9-dihydrofuro[3,4-b]quinoléin-1(3*H*)-one**

Le produit attendu est obtenu selon le procédé décrit dans l'Exemple 9 à partir de 3,4-méthylènedioxyaniline, de bromure de propyle et du composé décrit dans la Préparation A.
*Point de fusion : 230°C*

**EXEMPLE 12 : (±) 4-(N,N-Diméthyl-aminopropyl)-6,7-méthylènedioxy-9-(3,4,5-triméthoxyphényl)-4,9-dihydrofuro[3,4-b]quinoléin-1(3*H*)-one**

Le produit attendu est obtenu selon le procédé décrit dans l'Exemple 9 à partir de 3,4-méthylènedioxyaniline, de 3-chloro-1-diméthylaminopropane et du composé décrit dans la Préparation A.
*Point de fusion : 182°C*

**EXEMPLE 13 : (±) 4,9-Bis(3,4,5-triméthoxyphényl)-6,7-méthylènedioxy-4,9-dihydrofuro[3,4-b]quinoléin-1(3*H*)-one**

*Stade A : N-(3,4-Méthylènedioxyphényl)-3,4,5-triméthoxyaniline*

A 10 mmoles de 3,4-méthylènedioxyaniline en solution dans le toluène sont ajoutées 10 mmoles de 3,4,5-triméthoxyiodobenzène, 0,4 mmole d'acétate de palladium, 0,4 mmole de 2,2'-bis(diphénylphosphino)-1,1'-binaphtyle et 14 mmoles de *tert*-butylate de potassium. Après 15 minutes de chauffage à 100°C, le mélange réactionnel est ramené à température ambiante, puis du dichlorométhane et de l'eau sont ajoutés. La phase organique est ensuite séparée et distillée sous pression réduite. Le résidu est purifié par chromatographie sur silice en utilisant le dichlorométhane comme éluant pour conduire au produit attendu sous la forme d'une huile.
IR (film) : 3361, 2934, 1602, 1503, 1488, 1232, 1204, 1128, 1037, 930 et 813 $cm^{-1}$

*Stade B : (±) 4,9-Bis(3,4,5-triméthoxyphényl)-6,7-méthylènedioxy-4,9-dihydrofuro[3,4-b]quinoléin-1(3*H*)-one*

Le produit attendu est obtenu selon le procédé décrit dans le stade B de l'Exemple 1 à partir du composé obtenu dans le stade précédent et du composé décrit dans la Préparation A.
*Point de fusion : > 260°C*

| *Microanalyse élementaire : (hémihydrate)* | | | |
|---|---|---|---|
| | *%C* | *%H* | *%N* |
| *Calculé* | *62,93* | *5,28* | *2,44* |
| *Trouvé* | *62,73* | *5,11* | *2,21* |

**EXEMPLE 14 : (±) 6,7-Diméthoxy-9-(3,4,5-triméthoxyphényl)-4,9-dihydrofuro [3,4-b]quinoléin-1(3*H*)-one**

Le produit attendu est obtenu selon le procédé décrit dans le stade B de l'Exemple 1 à partir de 3,4-diméthoxyaniline et du composé décrit dans la Préparation A.
*Point de fusion : 218°C*

**EXEMPLE 15 : (±) 9-(3,5-Diméthoxy-4-hydroxyphényl)-4-méthyl-6,7-méthylènedioxy-4,9-dihydrofuro[3,4-b]quinoléin-1(3*H*)-one**

Le produit attendu est obtenu selon le procédé décrit dans le stade B de l'Exemple 1 à partir du composé décrit dans le stade A de l'Exemple 1 et du composé décrit dans la Préparation B.

*Point de fusion : 250°C*

| *Microanalyse élémentaire : (hydrate)* | | | |
|---|---|---|---|
| | *%C* | *%H* | *%N* |
| *Calculé* | *60,72* | *4,85* | *3,37* |
| *Trouvé* | *60,78* | *4,99* | *3,14* |

**EXEMPLE 16 : (±) 9-(3,5-Diméthoxy-4-hydroxyphényl)-6,7-méthylènedioxy-4-(4-nitrobenzyl)-4,9-dihydrofuro [3,4-b]quinoléin-1(3*H*)-one**

Le produit attendu est obtenu selon le procédé décrit dans le stade B de l'Exemple 1 à partir du composé décrit dans le stade A de l'Exemple 3 et du composé décrit dans la Préparation B.
*Point de fusion : 193°C*

| *Microanalyse élémentaire : (hémihydrate)* | | | |
|---|---|---|---|
| | *%C* | *%H* | *%N* |
| *Calculé* | *61,48* | *4,30* | *5,31* |
| *Trouvé* | *61,61* | *4,36* | *5,11* |

**EXEMPLE 17 : (±) 4-(4-Chlorobenzyl)-9-(3,5-diméthoxy-4-hydroxyphényl)-6,7-méthylènedioxy-4,9-dihydrofuro [3,4-b]quinoléin-1(3*H*)-one**

Le produit attendu est obtenu selon le procédé décrit dans l'Exemple 3 à partir de 3,4-méthylènedioxyaniline, de 4-chlorobenzaldéhyde et du composé décrit dans la Préparation B.
*Point de fusion : 252°C*

**EXEMPLE 18 : (±) 9-(3,5-Diméthoxy-4-hydroxyphényl)-4-(3-hydroxybenzyl)-6,7-méthylènedioxy-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de 3-hydroxybenzaldéhyde et du composé décrit dans la Préparation B.
*Point de fusion : 220°C*

| *Microanalyse élémentaire : ($C_{27}H_{23}NO_8$.1,5 $H_2O$)* | | | |
|---|---|---|---|
| | *%C* | *%H* | *%N* |
| *Calculé* | *62,79* | *5,07* | *2,71* |
| *Trouvé* | *62,44* | *4,89* | *2,75* |

**EXEMPLE 19 : (±) 9-(3,5-Diméthoxy-4-hydroxyphényl)-6,7-méthylènedioxy-4-(3,4-méthylènedioxybenzyl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de 3,4-méthylènedioxybenzaldéhyde et du composé décrit dans la Préparation B.
*Point de fusion : > 260°C*

| *Microanalyse élémentaire : (hémihydrate)* | | | |
|---|---|---|---|
| | *%C* | *%H* | *%N* |
| *Calculé* | *63,88* | *4,59* | *2,66* |
| *Trouvé* | *63,89* | *4,51* | *2,69* |

**EXEMPLE 20 : (±) 9-(3,5-Diméthoxy-4-hydroxyphényl)-6,7-méthylènedioxy-4-(3,4,5-triméthoxybenzyl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de 3,4,5-triméthoxybenzaldéhyde et du composé décrit dans la Préparation B.
*Point de fusion : 165°C*

| *Microanalyse élémentaire : ($C_{30}H_{29}NO_{10}$.2,5 $H_2O$)* | | | |
|---|---|---|---|
| | *%C* | *%H* | *%N* |
| *Calculé* | *59,21* | *5,63* | *2,30* |
| *Trouvé* | *59,22* | *5,51* | *2,34* |

**EXEMPLE 21 : (±) 4-(3,5-Diméthoxy-4-hydroxybenzyl)-9-(3,5-diméthoxy-4-hydroxyphényl)-6,7-méthylènedioxy-4,9-dihydrofuro[3,4-b] quinoléin-1(3*H*)-one**

Le produit attendu est obtenu selon le procédé décrit dans l'Exemple 3 à partir de 3,4-méthylènedioxyaniline, de 3,5-diméthoxy-4-hydroxybenzaldéhyde et du composé décrit dans la Préparation B.
*Point de fusion : 235°C*

| *Microanalyse élémentaire : (hémihydrate)* | | | |
|---|---|---|---|
| | *%C* | *%H* | *%N* |
| *Calculé* | *62,36* | *5,05* | *2,51* |
| *Trouvé* | *62,16* | *4,96* | *2,34* |

**EXEMPLE 22 : (±) 9-(3,5-Diméthoxy-4-hydroxyphényl)-4-(2-hydroxyéthyl)-6,7-méthylènedioxy-4,9-dihydrofuro [3,4-b]quinoléin-1(3*H*)-one**

Le produit attendu est obtenu selon le procédé décrit dans le stade B de l'Exemple 1 à partir du composé décrit dans le stade A de l'Exemple 9 et du composé décrit dans la Préparation B.
*Point de fusion : > 260°C*

| *Microanalyse élémentaire : (hémihydrate)* | | | |
|---|---|---|---|
| | *%C* | *%H* | *%N* |
| *Calculé* | *60,55* | *5,08* | *3,21* |
| *Trouvé* | *60,84* | *5,05* | *3,11* |

**EXEMPLE 23 : (±) 9-(3,5-Diméthoxy-4-hydroxyphényl)-6-méthyl-4,9-dihydrofuro [3,4-b]quinoléin-1(3*H*)-one**

Le produit attendu est obtenu selon le procédé décrit dans le stade B de l'Exemple 1 à partir de m-toluidine et du composé décrit dans la Préparation B.

**EXEMPLE 24 : (±) 6,7-Méthylènedioxy-9-(3-pyridyl)-4,9-dihydrofuro[3,4-b] quinoléin-1(3*H*)-one**

Le produit attendu est obtenu selon le procédé décrit dans le stade B de l'Exemple 1 à partir de 3,4-méthylènedioxyaniline et du composé décrit dans la Préparation C.
*Point de fusion : > 260°C*

| *Microanalyse élémentaire : (hémihydrate)* | | | |
|---|---|---|---|
| | *%C* | *%H* | *%N* |
| *Calculé* | *64,35* | *4,13* | *8,83* |
| *Trouvé* | *64,14* | *4,15* | *8,61* |

**EXEMPLE 25 : (±) 6,7-Ethylènedioxy-9-(3-méthoxy-4,5-méthylènedioxyphényl)-4-[(2-thiényl)-méthyl]-4,9-dihydrofuro[3,4-b]quinoléin-1(3*H*)-one**

Le produit attendu est obtenu selon le procédé décrit dans l'Exemple 3 à partir de 3,4-éthylènedioxyaniline, de thiophène-2-carboxaldéhyde et du composé décrit dans la Préparation D.

**EXEMPLE 26 : (±) 4-(Diméthylaminoéthylcarbonyl)-9-(3-méthoxy-4,5-méthylène dioxyphényl)-6,7-méthylènedioxy-4,9-dihydrofuro[3,4-b] quinoléin-1(3*H*)-one**

Le produit attendu est obtenu selon le procédé décrit dans l'Exemple 9 à partir de 3,4-méthylènedioxyaniline, de chlorure de 3-(diméthylamino)-propionyle et du composé décrit dans la Préparation D.

**EXEMPLE 27 : (±) 6-Méthoxy-9-(3,4,5-triméthoxyphényl)-4,9-dihydrofuro[3,4-b] quinoléin-1(3*H*)-one**

Le produit attendu est obtenu selon le procédé décrit dans le stade B de l'Exemple 1 à partir de 3-méthoxyaniline et du composé décrit dans la Préparation A.
*Point de fusion : > 260°C*

| *Microanalyse élémentaire :* | | | |
|---|---|---|---|
| | *%C* | *%H* | *%N* |
| *Calculé* | *65,79* | *5,52* | *3,65* |
| *Trouvé* | *65,80* | *5,60* | *3,50* |

**EXEMPLE 28 : (±) 6,7,8-Triméthoxy-9-(3,4,5-triméthoxyphényl)-4,9-dihydrofuro [3,4-b]quinoléin-1(3*H*)-one**

Le produit attendu est obtenu selon le procédé décrit dans le stade B de l'Exemple 1 à partir de 3,4,5-triméthoxyaniline et du composé décrit dans la Préparation A.
*Point de fusion : > 260°C*

| *Microanalyse élémentaire :* | | | |
|---|---|---|---|
| | *%C* | *%H* | *%N* |
| *Calculé* | *61,05* | *5,79* | *3,09* |
| *Trouvé* | *61,19* | *6,02* | *3,00* |

**EXEMPLE 29 : (±) 6-Hydroxy-9-(3,4,5-triméthoxyphényl)-4,9-dihydrofuro[3,4-b] quinoléin-1(3*H*)-one**

Le produit attendu est obtenu selon le procédé décrit dans le stade B de l'Exemple 1 à partir de 3-aminophénol et du composé décrit dans la Préparation A.
*Point de fusion : > 260°C*

| *Microanalyse élémentaire : (0,25 $H_2O$)* | | | |
|---|---|---|---|
| | *%C* | *%H* | *%N* |
| *Calculé* | *64,25* | *5,26* | *3,74* |
| *Trouvé* | *64,41* | *5.24* | *3,62* |

**EXEMPLE 30 : (±) 6,8-Diméthoxy-9-(3,4,5-triméthoxyphényl)-4,9-dihydrofuro [3,4-b]quinoléin-1(3*H*)-one**

Le produit attendu est obtenu selon le procédé décrit dans le stade B de l'Exemple 1 à partir de 3,5-diméthoxyaniline et du composé décrit dans la Préparation A.
*Point de fusion : > 260°C*

| *Microanalyse élémentaire :* | | | |
|---|---|---|---|
| | *%C* | *%H* | *%N* |
| *Calculé* | *63,91* | *5,61* | *3,39* |
| *Trouvé* | *63,85* | *5,77* | *3,24* |

**EXEMPLE 31 : (±) 6,7-Méthylènedioxy-9-(2,3,4-triméthoxyphényl)-4,9-dihydrofuro [3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation E.
*Point de fusion : > 260°C*

| *Microanalyse élémentaire :* | | | |
|---|---|---|---|
| | *%C* | *%H* | *%N* |
| *Calculé* | *63,47* | *4,82* | *3,52* |
| *Trouvé* | *63,57* | *4,91* | *3,34* |

**EXEMPLE 32 : (±) 9-(3,4-Diméthoxyphényl)-6,7-méthylènedioxy-4,9-dihydrofuro [3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation F.
*Point de fusion : > 260°C*

| *Microanalyse élémentaire :* | | | |
|---|---|---|---|
| | *%C* | *%H* | *%N* |
| *Calculé* | *65,39* | *4,66* | *3,81* |
| *Trouvé* | *64,87* | *4,73* | *3,52* |

**EXEMPLE 33 : (±) 9-(3-Hydroxy-4-méthoxyphényl)-6,7-méthylènedioxy-4,9-dihydro furo[3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation G.
*Point de fusion : > 260°C*

| *Microanalyse élémentaire :* | | | |
|---|---|---|---|
| | *%C* | *%H* | *%N* |
| *Calculé* | *64,59* | *4,28* | *3,96* |
| *Trouvé* | *64,75* | *4,25* | *3,76* |

**EXEMPLE 34 : (±) 9-(4-Méthoxyphényl)-6,7-méthylènedioxy-4,9-dihydrofuro [3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation H.
*Point de fusion : > 260°C*

| *Microanalyse élémentaire :* | | | |
|---|---|---|---|
| | *%C* | *%H* | *%N* |
| *Calculé* | *67,65* | *4,48* | *4,15* |
| *Trouvé* | *67,98* | *4,43* | *3,95* |

**EXEMPLE 35 : (±) 9-(3-Méthoxyphényl)-6,7-méthylènedioxy-4,9-dihydrofuro[3,4-b] quinoléin-1(3*H*)-one**

Le produit attendu est obtenu selon le procédé décrit dans le stade B de l'Exemple 1 à partir de 3,4-méthylènedioxyaniline et du composé décrit dans la Préparation I.
*Point de fusion : > 260°C*

| Microanalyse élémentaire : | | | |
|---|---|---|---|
| | %C | %H | %N |
| Calculé | 67,65 | 4,48 | 4,15 |
| Trouvé | 67,48 | 4,49 | 3,97 |

**EXEMPLE 36 : (±) 6,7-Méthylènedioxy-9-phényl-4,9-dihydrofuro[3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation J.
*Point de fusion : > 260°C*

| Microanalyse élémentaire : | | | |
|---|---|---|---|
| | %C | %H | %N |
| Calculé | 70,35 | 4,26 | 4,56 |
| Trouvé | 70,28 | 4,18 | 4,38 |

**EXEMPLE 37 : (±) 9-(2-Furyl)-6,7-méthylènedioxy-4,9-dihydrofuro[3,4-b] quinoléin-1(3*H*)-one**

Le produit attendu est obtenu selon le procédé décrit dans le stade B de l'Exemple 1 à partir de 3,4-méthylènedioxyaniline et du composé décrit dans la Préparation K.
*Point de fusion :* > 260°C

| Microanalyse élémentaire : | | | |
|---|---|---|---|
| | %C | %H | %N |
| Calculé | 64,65 | 3,73 | 4,71 |
| Trouvé | 62,25 | 3,87 | 4,31 |

**EXEMPLE 38 : (±) 7-(3,4,5-Triméthoxyphényl)-7,11-dihydrobenzo[h]furo[3,4-b] quinoléin-8(10*H*)-one**

Le produit attendu est obtenu selon le procédé décrit dans le stade B de l'Exemple 1 à partir de 1-naphtylamine et du composé décrit dans la Préparation A.
*Point de fusion : > 260°C*

| Microanalyse élémentaire : (hémihydrate) | | | |
|---|---|---|---|
| | %C | %H | %N |
| Calculé | 69,89 | 5,34 | 3,39 |
| Trouvé | 70,28 | 5,40 | 3,31 |

**EXEMPLE 39 : (±) 11-(3,4,5-Triméthoxyphényl)-7,11-dihydrobenzo[f]furo[3,4-b] quinoléin-10(8*H*)-one**

Le produit attendu est obtenu selon le procédé décrit dans le stade B de l'Exemple 1 à partir de 2-naphtylamine et du composé décrit dans la Préparation A.
*Point de fusion : > 260°C*

| *Microanalyse élémentaire :* | | | |
|---|---|---|---|
| | *%C* | *%H* | *%N* |
| *Calculé* | *71,45* | *5,25* | *3,47* |
| *Trouvé* | *69,90* | *5,44* | *3,44* |

**EXEMPLE 40 : (±) 6,7-Méthylènedioxy-9-(3,4,5-triméthoxyphényl)-2,3,4,9-tétrahydrocyclopenta[b]quinoléin-1-one**

Le produit attendu est obtenu selon le procédé décrit dans le stade B de l'Exemple 1 à partir de 3,4-méthylènedioxyaniline et du composé décrit dans la Préparation L.
*Point de fusion : > 260°C*

| *Microanalyse élémentaire : (0,25 $H_2O$)* | | | |
|---|---|---|---|
| | *%C* | *%H* | *%N* |
| *Calculé* | *66,07* | *5,42* | *3,50* |
| *Trouvé* | *66,09* | *5,34* | *3,36* |

**EXEMPLE 41 : (±) 6,7-Méthylènedioxy-9-(3,4-méthylènedioxyphényl)-4,9-dihydro furo[3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation M.
*Point de fusion : > 260°C*

| *Microanalyse élémentaire :* | | | |
|---|---|---|---|
| | *%C* | *%H* | *%N* |
| *Calculé* | *64,96* | *3,73* | *3,99* |
| *Trouvé* | *65,12* | *4,19* | *3,75* |

**EXEMPLE 42 : (±)10-(3,4,5-Triméthoxyphényl)-1,6,7,10-tétrahydro-9*H*-furo[3,4-b] pyrrolo[2,3-f]quinoléin-9-one**

Le produit attendu est obtenu selon le procédé décrit dans le stade B de l'Exemple 1 à partir de 1*H*-indol-6-amine et du composé décrit dans la Préparation A.
*Point de fusion : > 260°C*

| *Microanalyse élémentaire : (0,75 $H_2O$)* | | | |
|---|---|---|---|
| | *%C* | *%H* | *%N* |
| *Calculé* | *65,10* | *5,34* | *6,90* |
| *Trouvé* | *65,25* | *5,23* | *6,50* |

**EXEMPLE 43 : (±) 4-Benzyl-6,7-méthylènedioxy-9-(3,4,5-triméthoxyphényl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de benzaldéhyde et du composé décrit dans la Préparation A.
*Point de fusion : 236°C*

| *Microanalyse élémentaire :* | | | |
|---|---|---|---|
| | *%C* | *%H* | *%N* |
| *Calculé* | *68,98* | *5,17* | *2,87* |

(suite)

| *Microanalyse élémentaire :* | | | |
|---|---|---|---|
| | *%C* | *%H* | *%N* |
| *Trouvé* | *68,95* | *5,27* | *2,83* |

**<u>EXEMPLE 44</u> : (±) 9-(4-Chlorophényl)-6,7-méthylènedioxy-4,9-dihydrofuro[3,4-b] quinoléin-1(3*H*)-one**

Le produit attendu est obtenu selon le procédé décrit dans le stade B de l'Exemple 1 à partir de 3,4-méthylènedioxyaniline et du composé décrit dans la Préparation N.
*Point de fusion: > 260°C*

| *Microanalyse élémentaire : (hémihydrate)* | | | |
|---|---|---|---|
| | *%C* | *%H* | *%N* |
| *Calculé* | *62,24* | *3,77* | *4,03* |
| *Trouvé* | *62,33* | *3,90* | *3,90* |

**<u>EXEMPLE 45</u> : (±) 6,7-Méthylènedioxy-9-(4-nitrophényl)-4,9-dihydrofuro[3,4-b] quinoléin-1(3*H*)-one**

Le produit attendu est obtenu selon le procédé décrit dans le stade B de l'Exemple 1 à partir de 3,4-méthylènedioxyaniline et du composé décrit dans la Préparation O.
*Point de fusion : > 260°C*

| *Microanalyse élémentaire : (0,25 $H_2O$)* | | | |
|---|---|---|---|
| | %C | %H | %N |
| *Calculé* | *60,59* | *3,53* | *7,85* |
| *Trouvé* | *60,86* | *3,57* | *7,62* |

**<u>EXEMPLE 46</u>: (±) 6,7-Méthylènedioxy-9-(3-nitrophényl)-4,9-dihydrofuro[3,4-b] quinoléin-1(3*H*)-one**

Le produit attendu est obtenu selon le procédé décrit dans le stade B de l'Exemple 1 à partir de 3,4-méthylènedioxyaniline et du composé décrit dans la Préparation P.
*Point de fusion : > 260°C*

| *Microanalyse élémentaire :* | | | |
|---|---|---|---|
| | *%C* | *%H* | *%N* |
| *Calculé* | *60,59* | *3,53* | *7,85* |
| *Trouvé* | *60,30* | *3,65* | *7,69* |

**<u>EXEMPLE 47</u> : (±) 9-(3,5-Diméthoxy-4-hydroxyphényl)-6,7-méthylènedioxy-4,9-dihydrofuro[3,4-b]quinoléin-1 (3*H*)-one**

Le produit attendu est obtenu selon le procédé décrit dans le stade B de l'Exemple 1 à partir de 3,4-méthylènedioxyaniline et du composé décrit dans la Préparation B.
*Point de fusion : > 260°C*

| *Microanalyse élémentaire : (0,25 $H_2O$)* | | | |
|---|---|---|---|
| | *%C* | *%H* | *%N* |
| *Calculé* | *61,93* | *4,55* | *3,61* |
| *Trouvé* | *61,93* | *4,59* | *3,34* |

**EXEMPLE 48 : (±) 9-(4-Diméthylaminophényl)-6,7-méthylènedioxy-4,9-dihydro furo[3,4-b]quinoléin-1(3*H*)-one**

Le produit attendu est obtenu selon le procédé décrit dans le stade B de l'Exemple 1 à partir de 3,4-méthylènedioxyaniline et du composé décrit dans la Préparation Q.
*Point de fusion : > 260°C*

| *Microanalyse élémentaire : (0,25 $H_2O$)* | | | |
|---|---|---|---|
| | *%C* | *%H* | *%N* |
| *Calculé* | *67,69* | *5,25* | *7,89* |
| *Trouvé* | *67,52* | *5,29* | *7,69* |

**EXEMPLE 49 : (±) 6,7-Ethylènedioxy-9-(3,4,5-triméthoxyphényl)-4,9-dihydrofuro [3,4-b]quinoléin-1(3*H*)-one**

Le produit attendu est obtenu selon le procédé décrit dans le stade B de l'Exemple 1 à partir de 3,4-éthylènedioxyaniline et du composé décrit dans la Préparation A.
*Point de fusion : > 260°C*

| *Microanalyse élémentaire : (0,25 $H_2O$)* | | | |
|---|---|---|---|
| | *%C* | *%H* | *%N* |
| *Calculé* | *63,53* | *5,21* | *3,37* |
| *Trouvé* | *63,56* | *5,18* | *3,39* |

**EXEMPLE 50 : (±) 4-Méthyl-6,7-méthylènedioxy-9-(4-pyridyl)-4,9-dihydrofuro [3,4-b]quinoléin-1(3*H*)-one**

Le produit attendu est obtenu selon le procédé décrit dans le stade B de l'Exemple 1 à partir du composé décrit dans le stade A de l'Exemple 1 et du composé décrit dans la Préparation R.
*Point de fusion : 255°C*

| *Microanalyse élémentaire : (monohydrate)* | | | |
|---|---|---|---|
| | *%C* | *%H* | *%N* |
| *Calculé* | *66,15* | *4,47* | *8,57* |
| *Trouvé* | *65,88* | *4,48* | *8,42* |

**EXEMPLE 51 : (±) 6,7-Méthylènedioxy-9-(2-pyridyl)-4,9-dihydrofuro[3,4-b] quinoléin-1(3*H*)-one**

Le produit attendu est obtenu selon le procédé décrit dans le stade B de l'Exemple 1 à partir de 3,4-méthylènedioxyaniline et du composé décrit dans la Préparation S.
*Point de fusion : > 260°C*

| *Microanalyse élémentaire :* | | | |
|---|---|---|---|
| | *%C* | *%H* | *%N* |
| *Calculé* | *66,34* | *3,92* | *9.08* |
| *Trouvé* | *66,68* | *4,12* | *8,91* |

**EXEMPLE 52 : (±) 13-(3,5-Diméthoxy-4-hydroxyphényl)-4,13-dihydronaphto[2,3-f] furo[3,4-b]quinoléin-1(3*H*)-one**

Le produit attendu est obtenu selon le procédé décrit dans le stade B de l'Exemple 1 à partir de 2-anthrylamine et du composé décrit dans la Préparation B.
*Point de fusion : > 260°C*

| *Microanalyse élementaire : ($CH_3OH$)* | | | |
|---|---|---|---|
| | *%C* | *%H* | *%N* |
| *Calculé* | *71,32* | *5,35* | *2,91* |
| *Trouvé* | *71,10* | *5,72* | *2,72* |

**EXEMPLE 53 : (±) 13-(3,4,5-Triméthoxyphényl)-4,13-dihydronaphto[2,3-f]furo [3,4-b]quinoléin-1(3*H*)-one**

Le produit attendu est obtenu selon le procédé décrit dans le stade B de l'Exemple 1 à partir de 2-anthrylamine et du composé décrit dans la Préparation A.
*Point de fusion : > 260° C*

| *Microanalyse élémentaire : (hémihydrate)* | | | |
|---|---|---|---|
| | *%C* | *%H* | *%N* |
| *Calculé* | *72,72* | *5,23* | *3,03* |
| *Trouvé* | *73,05* | *5,73* | *2,71* |

**EXEMPLE 54 : (±) 6,7-Méthylènedioxy-4-(4-pyridylméthyl)-9-(3,4,5-triméthoxyphényl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, d'isonicotinaldéhyde et du composé décrit dans la Préparation A.
*Point de fusion : 240° C*

| *Microanalyse élémentaire :* | | | |
|---|---|---|---|
| | *%C* | *%H* | *%N* |
| *Calculé* | *66,38* | *4,95* | *5,73* |
| *Trouvé* | *66,24* | *4,97* | *5,71* |

**EXEMPLE 55 : (±) 6,7-Méthylènedioxy-4-(2-pyridylméthyl)-9-(3,4,5-triméthoxy phényl)-4,9-dihydrofuro[3,4-b]quinoléin-1(3*H*)-one**

Le produit attendu est obtenu selon le procédé décrit dans l'Exemple 3 à partir de 3,4-méthylènedioxyaniline, de 2-pyridinecarboxaldéhyde et du composé décrit dans la Préparation A.
*Point de fusion : 258° C*

| *Microanalyse élémentaire :* | | | |
|---|---|---|---|
| | *%C* | *%H* | *%N* |
| *Calculé* | *66,38* | *4,95* | *5,73* |
| *Trouvé* | *66,25* | *5,01* | *5,72* |

**EXEMPLE 56 : (±) 7-(3,4,5-Triméthoxyphényl)-7,9,10,11-tétrahydro-8*H*-benzo[h] cyclopenta[b]quinoléin-8-one**

Le produit attendu est obtenu selon le procédé décrit dans le stade B de l'Exemple 1 à partir de 1-naphtylamine et du composé décrit dans la Préparation L.
*Point de fusion : 250° C*

| *Microanalyse élémentaire :* | | | |
|---|---|---|---|
| | *%C* | *%H* | *%N* |
| *Calculé* | *74,79* | *5,77* | *3,49* |
| *Trouvé* | *74,46* | *6,37* | *3,09* |

**EXEMPLE 57 : (±) 6-Méthoxy-9-(3,4,5-triméthoxyphényl)-2,3,4,9-tétrahydro-1*H*-cyclopenta[b]quinoléin-1-one**

Le produit attendu est obtenu selon le procédé décrit dans le stade B de l'Exemple 1 à partir de 3-méthoxyaniline et du composé décrit dans la Préparation L.
*Point de fusion : 254°C*

| Microanalyse élémentaire : (0,25 $H_2O$) | | | |
|---|---|---|---|
| | %C | %H | %N |
| Calculé | 68,47 | 6,14 | 3,63 |
| Trouvé | 68,41 | 6,30 | 3,43 |

**EXEMPLE 58 : (±) 6,7-Méthylènedioxy-4-(2-thiénylméthyl)-9-(3,4,5-triméthoxy phényl)-4,9-dihydrofuro[3,4-b] quinoléin-1(3*H*)-one**

Le produit attendu est obtenu selon le procédé décrit dans l'Exemple 3 à partir de 3,4-méthylènedioxyaniline, de thiophène-2-carboxaldéhyde et du composé décrit dans la Préparation A.
*Point de fusion : 230°C*

| Microanalyse élémentaire : (0,25 $H_2O$) | | | |
|---|---|---|---|
| | %C | %H | %N |
| Calculé | 62,70 | 4,75 | 2,81 |
| Trouvé | 62,83 | 4,58 | 2,77 |

**EXEMPLE 59 : (±) 6,7-Méthylènedioxy-4-(3-thiénylméthyl)-9-(3,4,5-triméthoxy phényl)-4,9-dihydrofuro[3,4-b] quinoléin-1(3*H*)-one**

Le produit attendu est obtenu selon le procédé décrit dans l'Exemple 3 à partir de 3,4-méthylènedioxyaniline, de thiophène-3-carboxaldéhyde et du composé décrit dans la Préparation A.
*Point de fusion : 238°C*

| Microanalyse élémentaire : | | | |
|---|---|---|---|
| | %C | %H | %N |
| Calculé | 63,27 | 4,70 | 2,84 |
| Trouvé | 63,12 | 4,70 | 2,71 |

**EXEMPLE 60 : (+) 4-Méthyl-6,7-méthylènedioxy-9-(3,4,5-triméthoxyphényl)-4,9-dihydrofuro[3,4-b]quinoléin-1 (3*H*)-one**

Le produit attendu est obtenu par séparation par chromatographie HPLC sur colonne chirale du mélange racémique de l'Exemple 1.

**EXEMPLE 61 : (-) 4-Méthyl-6,7-méthylènedioxy-9-(3,4,5-triméthoxyphényl)-4,9-dihydrofuro[3,4-b]quinoléin-1 (3*H*)-one**

Le produit attendu est obtenu par séparation par chromatographie HPLC sur colonne chirale du mélange racémique de l'Exemple 1.

**EXEMPLE 62 : (±) 6-(3,4,5-Triméthoxyphényl)-2,3,4,6,9,10-hexahydro-7*H*-furo [3,4-b]pyrano[3,2-g]quinoléin-7-one**

Le produit attendu est obtenu selon le procédé décrit dans le stade B de l'Exemple 1 à partir de 7-chromanamine et du composé décrit dans la Préparation A.

**EXEMPLE 63 : (±) 9-(3,4,5-Triméthoxyphényl)-2,3,6,9-tétrahydrodifuro[3,4-b:2,3-g] quinoléin-8(*5H*)-one**

Le produit attendu est obtenu selon le procédé décrit dans le stade B de l'Exemple 1 à partir de 2,3-dihydro-1-benzofuran-5-amine et du composé décrit dans la Préparation A.

**EXEMPLE 64 : (±) 9-Benzyl-6,7-méthylènedioxy-4,9-dihydrofuro[3,4-b]qinoléin-1(3*H*)-one**

A 10 mmoles de 3,4-méthylènedioxyaniline en solution dans l'éthanol sont ajoutées 10 mmoles de phénylacétaldéhyde et 10 mmoles d'acide tétronique, puis le mélange réactionnel est chauffé à reflux pendant la nuit. Après retour à température ambiante, le précipité obtenu est filtré puis lavé à l'éthanol pour conduire au produit attendu.

**EXEMPLE 65 : (±) 6,7-Méthylènedioxy-9-(3,4,5-triméthoxyphényl)-3,4,9,10-tétrahydro-1(2*H*)-acridinone**

Le produit attendu est obtenu selon le procédé décrit dans l'Exemple 64 à partir de 3,4-méthylènedioxyaniline, de 3,4,5-triméthoxybenzaldéhyde et de 1,3-cyclohexanedione.

**EXEMPLE 66 : (±) 6,7-Méthylènedioxy-9-(3,4,5-triméthoxyphényl)-4,9-dihydrothiéno[3,4-b]quinoléin-1(3*H*)-one**

Le produit attendu est obtenu selon le procédé décrit dans l'Exemple 64 à partir de 3,4-méthylènedioxyaniline, de 3,4,5-triméthoxybenzaldéhyde et d'acide thiotétronique.
*Point de fusion : > 260°C*

| *Microanalyse élémentaire :* | | | |
|---|---|---|---|
| | *%C* | *%H* | *%N* |
| *Calculé* | *61,00* | *4,63* | *3,39* |
| *Trouvé* | *60,84* | *4,66* | *3,55* |

**EXEMPLE 67 : (±) 7-( 3,4,5-Triméthoxyphényl)-7,11-dihydrobenzo[h]thiéno[3,4-b] quinoléin-8(10*H*)-one**

Le produit attendu est obtenu selon le procédé décrit dans l'Exemple 64 à partir de 1-naphtylamine, de 3,4,5-triméthoxybenzaldéhyde et d'acide thiotétronique.
*Point de fusion : > 260°C*

| *Microanalyse élémentaire :* | | | |
|---|---|---|---|
| | *%C* | *%H* | *%N* |
| *Calculé* | *68,13* | *5,19* | *3,27* |
| *Trouvé* | *68,48* | *5,60* | *3,02* |

**ETUDE PHARMACOLOGIQUE DES COMPOSES DE L'INVENTION**

**EXEMPLE 68 : Cytotoxicité in vitro**

Trois lignées cellulaires ont été utilisées :

- 1 leucémie murine, L1210,
- 1 carcinome pulmonaire humain, non à petites cellules, A549,
- 1 carcinome colique humain, HT29.

Les cellules sont cultivées dans du milieu RPMI 1640 complet contenant 10 % de sérum de veau foetal, 2 mM de glutamine, 50 unités/ml de pénicilline, 50 μg/ml de streptomycine et 10 mM d'Hepes, pH = 7,4. Les cellules sont réparties dans des microplaques et exposées aux composés cytotoxiques. Les cellules sont ensuite incubées pendant 2 jours (L1210) ou 4 jours (A549, HT29). Le nombre de cellules viables est ensuite quantifié par un essai colorimétrique, le Microculture Tetrazolium Assay (Cancer Res. 1987, 47, 939-942).
Les résultats sont exprimés en $IC_{50}$, concentration en cytotoxique qui inhibe à 50 % la prolifération des cellules traitées. A titre d'exemple, les composés des exemples 1, 8 et 10 présentent les $IC_{50}$ mentionnées dans le tableau ci-

dessous :

| | $IC_{50}$ nM | | |
|---|---|---|---|
| Composés testés | L1210 | A549 | HT29 |
| Exemple 1 | 53 | 102 | 104 |
| Exemple 8 | 62 | 147 | 153 |
| Exemple 10 | 12 | 22 | 18 |

**EXEMPLE 69 : Activité *in vivo* : activité antitumorale des composés sur la leucémie P388**

La lignée P388 (leucémie murine) a été fournie par le National Cancer Institute (Frederick USA). Les cellules tumorales ($10^6$ cellules) ont été inoculées au jour 0 dans la cavité péritonéale de souris BDF femelles (Iffa-Credo. France) pesant de 18 à 20 g (groupes de 6 animaux). Les produits ont été administrés au jour 1 (J1) par voie intrapéritonéale.

L'activité antitumorale est exprimée en % de T/C :

$$\% \ T/C = \frac{\text{Temps de survie médian des animaux traités}}{\text{Temps de survie médian des animaux contrôlés}} \times 100$$

A titre d'exemple, le composé de l'exemple 1 est actif à partir de la dose de 50 mg/kg et il peut permettre de doubler la survie des animaux traités. (T/C ≥ 200 %)

**EXEMPLE 70 : Composition pharmaceutique**

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

**Revendications**

1. Composé de formule (I) :

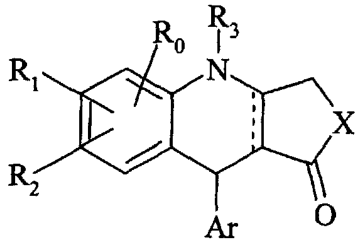

(I)

dans laquelle :

➢ ---- représente une liaison double,

➢ $R_0$ représente un atome d'hydrogène ou un groupement hydroxy ou alkoxy ($C_1$-$C_6$) linéaire ou ramifié,

➢ $R_1$ et $R_2$, identiques ou différents, représentent chacun :

- un atome d'hydrogène,
- un atome d'halogène,
- un groupement alkyle ($C_1$-$C_6$) linéaire ou ramifié,
- un groupement alkoxy ($C_1$-$C_6$) linéaire ou ramifié,
- un groupement hydroxy,
- un groupement polyhalogénoalkyle ($C_1$-$C_6$) linéaire ou ramifié,
- un groupement nitro,
- un groupement amino éventuellement substitué par un ou deux groupements alkyle ($C_1$-$C_6$) linéaire ou ramifié,
- un groupement de formule

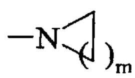

dans laquelle m représente un entier tel que $1 \leq m \leq 4$,
ou forment ensemble avec les atomes de carbone qui les portent un groupement mono-ou bicyclique de 5 à 12 chaînons, aromatique ou non-aromatique, contenant éventuellement 1 ou 2 hétéroatomes choisis parmi O, S et N,

➢ $R_3$ représente un atome d'hydrogène ou un groupement de formule $R_4$ dans laquelle $R_4$ représente :

- un groupement aryle,
- un groupement hétéroaryle,
- un groupement cycloalkyle ($C_3$-$C_8$),
- un groupement alkyle ($C_1$-$C_6$) linéaire ou ramifié éventuellement substitué par un groupement aryle, par un groupement hétéroaryle, par un groupement hydroxy, par un groupement alkoxy ($C_1$-$C_6$) linéaire ou ramifié, ou par un groupement de formule $NR_5R_6$ dans laquelle $R_5$ et $R_6$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté,
- ou un groupement de formule $COR_7$, dans laquelle $R_7$ représente un groupement:
  - aryle,
  - alkyle ($C_1$-$C_6$) linéaire ou ramifié (éventuellement substitué par un groupement de formule $NR_8R_9$ dans laquelle $R_8$ et $R_9$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté),
  - amino éventuellement substitué par un ou plusieurs groupements aryle, hétéroaryle, ou alkyle ($C_1$-$C_6$) linéaire ou ramifié éventuellement substitué par un groupement de formule $NR_8R_9$ dans laquelle $R_8$ et $R_9$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui les portent un hétérocycle azoté,
  - ou $OR_{10}$ dans laquelle $R_{10}$ représente un atome d'hydrogène ou un groupement aryle, ou alkyle ($C_1$-$C_6$) linéaire ou ramifié éventuellement substitué par un groupement de formule $NR_8R_9$ dans laquelle $R_8$ et $R_9$, identiques ou différents, représentent chacun un groupement alkyle ($C_1$-$C_6$) linéaire ou ramifié, hydroxyalkyle ($C_1$-$C_6$) linéaire ou ramifié ou forment ensemble avec l'atome d'azote qui

les portent un hétérocycle azoté,

➢ X représente un atome d'oxygène, de soufre ou un groupement $-CH_2-$ ou $-CH_2-CH_2-$,

➢ Ar représente un groupement aryle, hétéroaryle ou arylalkyle ($C_1$-$C_6$) linéaire ou ramifié, leurs isomères optiques, leurs hydrates, leurs solvates ainsi que leurs sels d'addition à un acide pharmaceutiquement acceptable, à l'exclusion de la 7-chloro-9-phényl-4,9-dihydrothiéno[3,4-b]quinoléin-1(3*H*)-one, de la 7-chloro-4-méthyl-9-phényl-4,9dihydrothiéno[3,4-b]quinoléin-1(3*H*)-one, de la 7-chloro-4-[2-(diéthylamino)-éthyl]-9phényl-4,9-dihydrothiéno[3,4-b]quinoléin-1(3*H*)-one, de la 9-phényl-3,4,9,10-tétrahydro-1(2*H*)-acridinone, de la 9-(2-thiényl)-3,4,9,10-tétrahydro-1(2*H*)-acridinone et de la 9-(4-méthylphényl)-3,4,9,10-tétrahydro-1 (2*H*)-acridinone, étant entendu que par groupement aryle, on entend phényle, biphényle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle ($C_1$-$C_6$) linéaire ou ramifié, hydroxy, alkoxy ($C_1$-$C_6$) linéaire ou ramifié, polyhalogénoalkyle ($C_1$-$C_6$) linéaire ou ramifié, amino (substitué éventuellement par un ou plusieurs groupements alkyle ($C_1$-$C_6$) linéaire ou ramifié), nitro, acyle ($C_1$-$C_6$) linéaire ou ramifié ou alkylènedioxy ($C_1$-$C_2$), par groupement hétéroaryle, on entend un groupement aromatique mono- ou bicyclique de 5 à 12 chaînons contenant un, deux ou trois hétéroatomes choisis parmi oxygène, azote ou soufre étant entendu que l'hétéroaryle peut être éventuellement substitué par un ou plusieurs atomes ou groupements, identiques ou différents, choisis parmi les atomes d'halogène et les groupements alkyle ($C_1$-$C_6$) linéaire ou ramifié, hydroxy, alkoxy ($C_1$-$C_6$) linéaire ou ramifié, polyhalogénoalkyle ($C_1$-$C_6$) linéaire ou ramifié, ou amino (substitué éventuellement par un ou plusieurs groupements alkyle ($C_1$-$C_6$) linéaire ou ramifié), et par hétérocycle azoté, on entend un groupement monocyclique saturé de 5 à 7 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre.

**2.** Composé de formule (I) selon la revendication 1 tel que $R_0$ représente un atome d'hydrogène.

**3.** Composé de formule (I) selon l'une quelconque des revendications 1 ou 2 tel que $R_1$ et $R_2$, identiques ou différents, représentent chacun :

- un atome d'hydrogène,
- un atome d'halogène,
- un groupement alkyle ($C_1$-$C_6$) linéaire ou ramifié,
- un groupement alkoxy ($C_1$-$C_6$) linéaire ou ramifié,
- un groupement hydroxy,
- un groupement polyhalogénoalkyle ($C_1$-$C_6$) linéaire ou ramifié,
- un groupement nitro,
- un groupement amino éventuellement substitué par un ou deux groupements alkyle ($C_1$-$C_6$) linéaire ou ramifié, ou
- un groupement de formule

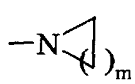

dans laquelle m représente un entier tel que $1 \leq m \leq 4$.

**4.** Composé de formule (I) selon l'une quelconque des revendications 1 ou 2 tel que $R_1$ et $R_2$ forment ensemble avec les atomes de carbone qui les portent un groupement mono- ou bicyclique de 5 à 12 chaînons, aromatique ou non-aromatique, contenant éventuellement 1 ou 2 hétéroatomes choisis parmi O, S et N.

**5.** Composé de formule (I) selon la revendication 4 tel que $R_1$ et $R_2$ forment ensemble avec les atomes de carbone qui les portent un groupement de formule $G_3$ ou $G_4$ :

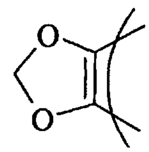
G3

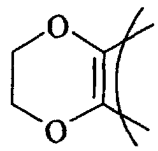
G4

6. Composé de formule (I) selon la revendication 4 tel que $R_1$ et $R_2$ forment ensemble avec les atomes de carbone qui les portent un groupement phénylène.

7. Composé de formule (I) selon l'une quelconque des revendications 1 à 6 tel que X représente un atome d'oxygène.

8. Composé de formule (I) selon l'une quelconque des revendications 1 à 6 tel que X représente un atome de soufre.

9. Composé de formule (I) selon l'une quelconque des revendications 1 à 6 tel que X représente un groupement $-CH_2-$ ou $-CH_2-CH_2-$.

10. Composé de formule (I) selon l'une quelconque des revendications 1 à 9 tel que Ar représente un groupement aryle.

11. Composé de formule (I) selon l'une quelconque des revendications 1 à 9 tel que Ar représente un groupement hétéroaryle.

12. Composé de formule (I) selon la revendication 10 tel que Ar représente un groupement phényle.

13. Composé de formule (I) selon la revendication 1 qui est la (±) 4-méthyl-6,7-méthylènedioxy-9-(3,4,5-triméthoxy-phényl)-4,9-dihydrofuro[3,4-b]quinoléin-1(3*H*)-one, ainsi que ses isomères optiques, ses hydrates et ses solvates.

14. Composé de formule (I) selon la revendication 1 qui est la (±) 6,7-méthylènedioxy-9-(3,4,5-triméthoxyphényl)-4,9-dihydrofuro[3,4-b]quinoléin-1(3*H*)-one, ses isomères optiques, ses hydrates, ses solvates ainsi que ses sels d'addition à un acide pharmaceutiquement acceptable.

15. Composé de formule (I) selon la revendication 1 qui est la (±) 6-méthoxy-9-(3,4,5-triméthoxyphényl)-4,9-dihydrofuro[3,4-b]quinoléin-1(3*H*)-one, ses isomères optiques, ses hydrates, ses solvates ainsi que ses sels d'addition à un acide pharmaceutiquement acceptable.

16. Composé de formule (I) selon la revendication 1 qui est la (±) 7-(3,4,5-triméthoxyphényl) 7,11-dihydrobenzo[h]furo [3,4-b]quinoléin-8(10*H*)-one, ses isomères optiques, ses hydrates, ses solvates ainsi que ses sels d'addition à un acide pharmaceutiquement acceptable.

17. Composé de formule (I) selon la revendication 1 qui est la (±)6,7-méthylènedioxy-9-(3,4,5-triméthoxyphényl)-2,3,4,9-tétrahydrocyclopenta[b]quinoléin-1-one, ses isomères optiques, ses hydrates, ses solvates ainsi que ses sels d'addition à un acide pharmaceutiquement acceptable.

18. Composé de formule (I) selon la revendication 1 qui est la (±) 6,7-éthylènedioxy-9-(3,4,5-triméthoxyphényl)-4,9-dihydrofuro[3,4-b]quinoléin-1(3*H*)-one, ses isomères optiques, ses hydrates, ses solvates ainsi que ses sels d'addition à un acide pharmaceutiquement acceptable.

19. Composé de formule (I) selon la revendication 1 qui est la (±) 7-(3,4,5-triméthoxy-phényl)-7,9,10,11-tétrahydro-8*H*-benzo[h]cyclopenta[b]quinoléin-8-one, ses isomères

20. Procédé de préparation des composés de formule (I) selon la revendication 1 **caractérisé en ce que** l'on met en réaction un composé de formule (II) :

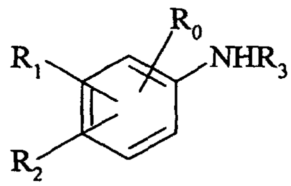

(II)

dans laquelle $R_0$, $R_1$, $R_2$ et $R_3$ ont la même signification que dans la formule (I), avec un composé de formule (III) :

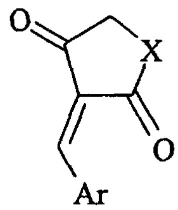

(III)

dans laquelle X et Ar ont la même signification que dans la formule (I),
pour conduire au composé de formule (I),
que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

**21.** Procédé de préparation des composés de formule (I) selon la revendication 1 **caractérisé en ce que** l'on met en réaction dans le même pot un composé de formule (II) :

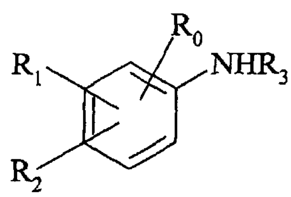

(II)

dans laquelle $R_0$, $R_1$, $R_2$ et $R_3$ ont la même signification que dans la formule (I),
un composé de formule (VIII) :

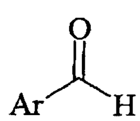

(VIII)

dans laquelle Ar a la même signification que dans la formule (I),
et un composé de formule (IX) :

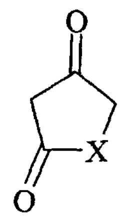

(IX)

dans laquelle X a la même signification que dans la formule (I), pour conduire au composé de formule (I), que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

**22.** Composition pharmaceutique contenant comme principe actif un composé selon l'une quelconque des revendications 1 à 19, seul ou en combinaison avec un ou plusieurs véhicules inertes, non toxiques et pharmaceutiquement acceptables.

**23.** Composition pharmaceutique selon la revendication 22 utile pour la fabrication de médicaments anticancéreux.

**Claims**

**1.** Compound of formula (I) :

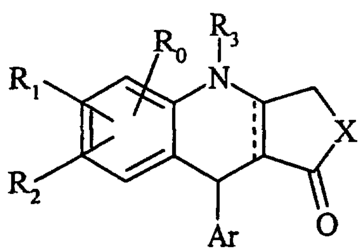

(I)

wherein:

➢ ---- represents a double bond,

➢ $R_0$ represents a hydrogen atom, a hydroxy group or a linear or branched ($C_1$-$C_6$)alkoxy group,

➢ $R_1$ and $R_2$, which are identical or different, each represents :

- a hydrogen atom,
- a halogen atom,
- a linear or branched ($C_1$-$C_6$)alkyl group,
- a linear or branched ($C_1$-$C_6$)alkoxy group,
- a hydroxy group,
- a linear or branched ($C_1$-$C_6$)polyhaloalkyl group,
- a nitro group,
- an amino group optionally substituted by one or two linear or branched ($C_1$-$C_6$)-alkyl groups,

a group of formula

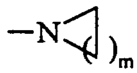

wherein m represents an integer such that $1 \leq m \leq 4$,
or form together with the carbon atoms carrying them an aromatic or non-aromatic, mono- or bi-cyclic group having from 5 to 12 ring members, optionally containing 1 or 2 hetero atoms selected from O, S and N,

➢ $R_3$ represents a hydrogen atom or a group of formula $R_4$ wherein $R_4$ represents :

- an aryl group,
- a heteroaryl group,
- a ($C_3$-$C_8$)cycloalkyl group,
- a linear or branched ($C_1$-$C_6$)alkyl group optionally substituted by an aryl group, by a heteroaryl group, by a hydroxy group, by a linear or branched ($C_1$-$C_6$)alkoxy group, or by a group of formula $NR_5R_6$ wherein $R_5$ and $R_6$, which are identical or different, each represents a linear or branched ($C_1$-$C_6$)alkyl group or a linear or branched ($C_1$-$C_6$)hydroxyalkyl group, or form together with the nitrogen atom carrying them a nitrogen heterocycle,
- or a group of formula $COR_7$, wherein $R_7$ represents one of the following groups :
    - aryl,
    - linear or branched ($C_1$-$C_6$)alkyl (optionally substituted by a group of formula $NR_8R_9$ wherein $R_8$ and $R_9$, which are identical or different, each represents a linear or branched ($C_1$-$C_6$)alkyl group or a linear or branched ($C_1$-$C_6$)hydroxyalkyl group, or form together with the nitrogen atom carrying them a nitrogen heterocycle),
    - amino optionally substituted by one or more groups aryl, heteroaryl, or linear or branched ($C_1$-$C_6$) alkyl optionally substituted by a group of formula $NR_8R_9$ wherein $R_8$ and $R_9$, which are identical or different, each represents a linear or branched ($C_1$-$C_6$)alkyl group or a linear or branched ($C_1$-$C_6$)-hydroxyalkyl group, or form together with the nitrogen atom carrying them a nitrogen heterocycle,
    - or $OR_{10}$ wherein $R_{10}$ represents a hydrogen atom or an aryl group, or linear or branched ($C_1$-$C_6$)alkyl optionally substituted by a group of formula $NR_8R_9$ wherein $R_8$ and $R_9$, which are identical or different, each represents a linear or branched ($C_1$-$C_6$)alkyl group or a linear or branched ($C_1$-$C_6$)-hydroxyalkyl group, or form together with the nitrogen atom carrying them a nitrogen heterocycle,

➢ X represents an oxygen atom, a sulphur atom or a $-CH_2-$ or $-CH_2-CH_2-$ group,

➢ Ar represents an aryl, heteroaryl or aryl-($C_1$-$C_6$)alkyl group in which alkyl is linear or branched,

their isomers, their hydrates, their solvates and also addition salts thereof with a pharmaceutically acceptable acid, with the exclusion of 7-chloro-9-phenyl-4,9-dihydrothieno[3,4-b]quinolin-1(3*H*)-one, 7-chloro-4-methyl-9-phenyl-4,9-dihydrothieno[3,4-b]quinolin-1(3*H*)-one, 7-chloro-4-[2-(di-ethylamino)ethyl]-9-phenyl-4,9-dihydrothieno[3,4-b]quinolin-1(3*H*)-one, 9-phenyl-3,4,9,10-tetrahydro-1(2*H*)-acridinone, 9-(2-thienyl)-3,4,9,10-tetrahydro-1(2*H*)-acridinone and 9-(4-methylphenyl)- 3,4,9,10-tetrahydro-1(2*H*)-acridinone,
wherein aryl is to be understood as phenyl, biphenyl, naphthyl or tetrahydronaphthyl, each of those groups being optionally substituted by one or more identical or different atoms or groups selected from halogen atoms and the groups linear or branched ($C_1$-$C_6$)alkyl, hydroxy, linear or branched ($C_1$-$C_6$)alkoxy, linear or branched ($C_1$-$C_6$) polyhaloalkyl, amino (optionally substituted by one or more linear or branched ($C_1$-$C_6$)alkyl groups), nitro, linear or branched ($C_1$-$C_6$)acyl and ($C_1$-$C_2$)alkylenedioxy,
heteroaryl is to be understood as an aromatic, mono- or bi-cyclic group having from 5 to 12 ring members and containing one, two or three hetero atoms selected from oxygen, nitrogen and sulphur, wherein the heteroaryl group may be optionally substituted by one or more identical or different atoms or groups selected from halogen atoms and the groups linear or branched ($C_1$-$C_6$)alkyl, hydroxy, linear or branched ($C_1$-$C_6$)alkoxy, linear or branched

$(C_1-C_6)$polyhaloalkyl, or amino (optionally substituted by one or more linear or branched $(C_1-C_6)$alkyl groups), and a nitrogen heterocycle is to be understood as a saturated monocyclic group having from 5 to 7 ring members and containing one, two or three hetero atoms, one of which is nitrogen while the additional hetero atom(s) optionally present is/are selected from oxygen, nitrogen or sulphur.

**2.** Compound of formula (I) according to claim 1, wherein $R_0$ represents a hydrogen atom.

**3.** Compound of formula (I) according to either claim 1 or claim 2, wherein $R_1$ and $R_2$, which are identical or different, each represents :

- a hydrogen atom,
- a halogen atom,
- a linear or branched $(C_1-C_6)$alkyl group,
- a linear or branched $(C_1-C_6)$alkoxy group,
- a hydroxy group,
- a linear or branched $(C_1-C_6)$polyhaloalkyl group,
- a nitro group,
- an amino group optionally substituted by one or two linear or branched $(C_1-C_6)$-alkyl groups, or
- a group of formula

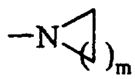

wherein m represents an integer such that $1 \leq m \leq 4$

**4.** Compound of formula (I) according to either claim 1 or claim 2, wherein $R_1$ and $R_2$ form together with the carbon atoms carrying them an aromatic or non-aromatic, mono- or bi-cyclic group having from 5 to 12 ring members and optionally containing 1 or 2 hetero atoms selected from O, S and N.

**5.** Compound of formula (I) according to claim 4, wherein $R_1$ and $R_2$ form together with the carbon atoms carrying them a group of formula $G_3$ or $G_4$ :

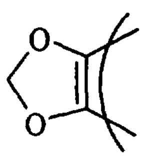

$G_3$

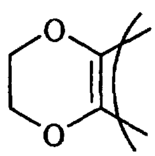

$G_4$

**6.** Compound of formula (I) according to claim 4, wherein $R_1$ and $R_2$ form together with the carbon atoms carrying them a phenylene group.

**7.** Compound of formula (I) according to any one of claims 1 to 6, wherein X represents an oxygen atom.

**8.** Compound of formula (I) according to any one of claims 1 to 6, wherein X represents a sulphur atom.

**9.** Compound of formula (I) according to any one of claims 1 to 6, wherein X represents a $-CH_2-$ or $-CH_2-CH_2-$ group.

**10.** Compound of formula (I) according to any one of claims 1 to 9, wherein Ar represents an aryl group.

**11.** Compound of formula (I) according to any one of claims 1 to 9, wherein Ar represents a heteroaryl group.

**12.** Compound of formula (I) according to claim 10, wherein Ar represents a phenyl group.

**13.** Compound of formula (I) according to claim 1 that is (±)-4-methyl-6,7-methylenedioxy-9-(3,4,5-trimethoxyphenyl)-4,9-dihydrofuro[3,4-b]quinolin-1(3*H*)-one, and also its optical isomers, its hydrates and its solvates.

**14.** Compound of formula (I) according to claim 1 that is (±)-6,7-methylenedioxy-9-(3,4,5-trimethoxyphenyl)-4,9-dihydrofuro[3,4-b]quinolin-1(3*H*)-one, its optical isomers, its hydrates, its solvates, and also addition salts thereof with a pharmaceutically acceptable acid.

**15.** Compound of formula (I) according to claim 1 that is (±)-6-methoxy-9-(3,4,5-trimethoxyphenyl)-4,9-dihydrofuro [3,4-b]quinolin-1(3*H*)-one, its optical isomers, its hydrates, its solvates, and also addition salts thereof with a pharmaceutically acceptable acid.

**16.** Compound of formula (I) according to claim 1 that is (±)-7-(3,4,5-trimethoxyphenyl)-7,11-dihydrobenzo[h]furo [3,4-b]quinolin-8(10*H*)-one, its optical isomers, its hydrates, its solvates, and also addition salts thereof with a pharmaceutically acceptable acid.

**17.** Compound of formula (I) according to claim 1 that is (±)-6,7-methylenedioxy-9-(3,4,5-trimethoxyphenyl)-2,3,4,9-tetrahydrocyclopenta[b]quinolin-1-one, its optical isomers, its hydrates, its solvates, and also addition salts thereof with a pharmaceutically acceptable acid.

**18.** Compound of formula (I) according to claim 1 that is (±)-6,7-ethylenedioxy-9-(3,4,5-trimethoxyphenyl)-4,9-dihydrofuro[3,4-b]quinolin-1(3*H*)-one, its optical isomers, its hydrates, its solvates, and also addition salts thereof with a pharmaceutically acceptable acid.

**19.** Compound of formula (I) according to claim 1 that is (±)-7-(3,4,5-trimethoxyphenyl)-7,9,10,11-tetrahydro-8*H*-benzo [h]cyclopenta[b]quinolin-8-one, its optical isomers, its hydrates, its solvates, and also its addition salts thereof with a pharmaceutically acceptable acid.

**20.** Process for the preparation of the compounds of formula (I) according to claim 1, **characterised in that** a compound of formula (II) :

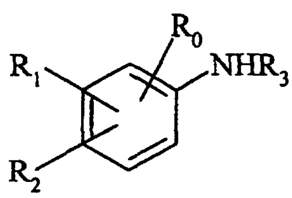

(II)

wherein $R_0$, $R_1$, $R_2$ and $R_3$ have the same meanings as for formula (I),
is reacted with a compound of formula (III) :

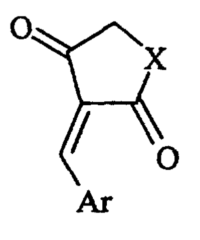

(III)

wherein X and Ar have the same meanings as for formula (I),
to yield a compound of formula (I),
which is purified, if necessary, according to a conventional purification technique, is separated, if desired, into its optical isomers according to a conventional separation technique, and is converted, if desired, into addition salts thereof with a pharmaceutically acceptable acid.

**21.** Process for the preparation of the compounds of formula (I) according to claim 1, **characterised in that** there are

reacted in the same pot a compound of formula (II) :

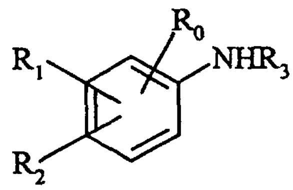

(II)

wherein $R_0$, $R_1$, $R_2$ and $R_3$ have the same meanings as for formula (I), a compound of formula (VIII) :

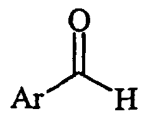

(VIII)

wherein Ar has the same meanings as for formula (I), and a compound of formula (IX) :

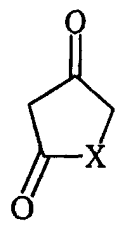

(IX)

wherein X has the same meanings as for formula (I), to yield a compound of formula (I), which is purified, if necessary, according to a conventional purification technique, is separated, if desired, into its optical isomers according to a conventional separation technique, and is converted, if desired, into addition salts thereof with a pharmaceutically acceptable acid.

**22.** Pharmaceutical composition comprising as active ingredient a compound according to any one of claims 1 to 19, alone or in combination with one or more pharmaceutically acceptable, inert, non-toxic carriers.

**23.** Pharmaceutical composition according to claim 22 for use in the manufacture of anticancer medicaments.

**Patentansprüche**

**1.** Verbindung der Formel (I):

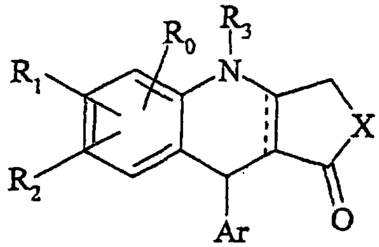

(I)

in der:

- ---- eine Doppelbindung darstellt,

- $R_0$ ein Wasserstoffatom oder eine Hydroxygruppe oder eine geradkettige oder verzweigte ($C_1$-$C_6$)-Alkoxygruppe bedeutet,

- $R_1$ und $R_2$, die gleichartig oder verschieden sind, jeweils:

  - ein Wasserstoffatom,
  - ein Halogenatom,
  - eine geradkettige oder verzweigte ($C_1$-$C_6$)-Alkylgruppe,
  - eine geradkettige oder verzweigte ($C_1$-$C_6$)-Alkoxygruppe,
  - eine Hydroxygruppe,
  - eine geradkettige oder verzweigte ($C_1$-$C_6$)-Polyhalogenalkylgruppe,
  - eine Nitrogruppe,
  - eine Aminogruppe, die gegebenenfalls durch eine oder zwei geradkettige oder verzweigte ($C_1$-$C_6$)-Alkylgruppen substituiert ist,
  - eine Gruppe der Formel

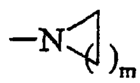

  in der m eine ganze Zahl mit Werten von $1 \leq m \leq 4$ darstellt,
  bedeuten oder gemeinsam mit dem sie tragenden Kohlenstoffatom eine monooder bicyclische, aromatische oder nichtaromatische Gruppe mit 5 bis 12 Kettengliedern, die gegebenenfalls ein oder zwei Heteroatome ausgewählt aus O, S und N enthält, bilden,

- $R_3$ ein Wasserstoffatom oder eine Gruppe der Formel $R_4$ bedeutet, worin $R_4$:

  - • eine Arylgruppe,
  - • eine Heteroarylgruppe,
  - • eine ($C_3$-$C_8$)-Cycloalkylgruppe.
  - • eine geradkettige oder verzweigte ($C_1$-$C_6$)-Alkylgruppe, die gegebenenfalls durch eine Arylgruppe, eine Heteroarylgruppe, eine Hydroxygruppe, eine geradkettige oder verzweigte ($C_1$-$C_6$)-Alkoxygruppe oder eine Gruppe der Formel $NR_5R_6$, worin $R_5$ und $R_6$, die gleichartig oder verschieden sind, jeweils eine geradkettige oder verzweigte ($C_1$-$C_6$)-Alkylgruppe, eine geradkettige oder verzweigte ($C_1$-$C_6$)-Hydroxyalkylgruppe darstellen oder gemeinsam mit dem sie tragenden Stickstoffatom einen Stickstoff-haltigen Heterocyclus bilden,
  - • oder eine Gruppe der Formel $COR_7$, worin $R_7$:

    - eine Arylgruppe,
    - eine geradkettige oder verzweigte ($C_1$-$C_6$)-Alkylgruppe (die gegebenenfalls durch eine Gruppe der Formel $NR_8R_9$ substituiert ist, in der $R_8$ und $R_9$, die gleichartig oder verschieden sind, jeweils eine geradkettige oder verzweigte ($C_1$-$C_6$)-Alkylgruppe oder eine geradkettige oder verzweigte ($C_1$-$C_6$) -Hydroxyalkylgruppe darstellen oder gemeinsam mit dem sie tragenden Stickstoffatom einen Stickstoff-haltigen Heterocyclus bilden),
    - eine Aminogruppe, die gegebenenfalls substituiert ist durch eine oder mehrere Arylgruppen, Heteroarylgruppen oder geradkettige oder verzweigte ($C_1$-$C_6$)-Alkylgruppen, die gegebenenfalls durch eine Gruppe der Formel $NR_8R_9$ substituiert ist, in der $R_8$ und $R_9$, die gleichartig oder verschieden sind, jeweils eine geradkettige oder verzweigte ($C_1$-$C_6$)-Alkylgruppe, eine geradkettige oder verzweigte ($C_1$-$C_6$)-Hydroxyalkylgruppe darstellen oder gemeinsam mit dem sie tragenden Stickstoffatom einen Stickstoff-haltigen Heterocyclus bilden,
    - oder eine Gruppe $OR_{10}$, in der $R_{10}$ ein Wasserstoffatom oder eine Arylgruppe, oder eine geradkettige oder verzweigte ($C_1$-$C_6$)-Alkylgruppe, die gegebenenfalls durch eine Gruppe der Formel $NR_8R_9$ substituiert ist, in der $R_8$ und $R_9$, die gleichartig oder verschieden sind, jeweils eine geradkettige oder

verzweigte ($C_1$-$C_6$)-Alkylgruppe oder eine geradkettige oder verzweigte ($C_1$-$C_6$)-Hydroxyalkylgruppe darstellen oder gemeinsam mit dem sie tragenden Stickstoffatom einen Stickstoff-haltigen Heterocylcus bilden, darstellt,
bedeutet;

➢ X ein Sauerstoffatom, ein Schwefelatom oder eine Gruppe $-CH_2-$ oder $-CH_2-CH_2-$ darstellt, und

➢ Ar eine Arylgruppe, Heteroarylgruppe oder geradkettige oder verzweigte Aryl-($C_1$-$C_6$)-alkylgruppe bedeutet,

deren optische Isomere, deren Hydrate, deren Solvate sowie deren Additionssalze mit einer pharmazeutisch annehmbaren Säure,
mit Ausnahme von 7-Chlor-9-phenyl-4,9-dihydrothieno[3,4-b]chinolin-1(3*H*)-on, 7-Chlor-4-methyl-9-phenyl-4,9-dihydrothieno[3,4-b]chinolin-1(3*H*)-on, 7-Chlor-4-[2-(diethylamino)-ethyl]-9-phenyl-4,9-dihydrothieno[3,4-b]chinolin-1(3*H*)-on, 9-Phenyl-,3,4,9,10-tetrahydro-1(2*H*)-acridinon, 9-(2-Thienyl)-3,4,9,10-tetrahydro-1(2*H*)-acridinon und 9-(4-Methylphenyl)-3,4,9,10-tetrahydro-1(2*H*)-acridinon, mit der Maßgabe, daß man unter einer Arylgruppe Phenyl-, Biphenyl, Naphthyl oder Tetrahydronaphthyl versteht, wobei jede dieser Gruppen gegebenenfalls durch ein oder mehrere gleichartige oder verschiedene Atome oder Gruppen ausgewählt aus Halogenatomen und geradkettigen oder verzweigten ($C_1$-$C_6$)-Alkylgruppen, Hydroxygruppen, geradkettigen oder verzweigten ($C_1$-$C_6$)-Alkoxygruppen, geradkettigen oder verzweigten ($C_1$-$C_6$)-Polyhalogenalkylgruppen, Aminogruppen (gegebenenfalls substituiert durch eine oder mehrere geradkettige oder verzweigte ($C_1$-$C_6$)-Alkylgruppen), Nitrogruppen, geradkettigen oder verzweigten ($C_1$-$C_6$)-Acylgruppen und ($C_1$-$C_2$)-Alkylendioxygruppen substituiert ist,
man unter einer Heteroarylgruppe eine aromatische, mono- oder bicyclische Gruppe mit 5 bis 12 Kettengliedern, die eins, zwei oder drei Heteroatome ausgewählt aus Sauerstoff, Stickstoff oder Schwefel enthält, versteht, mit der Maßgabe, daß Heteroaryl gegebenenfalls durch ein oder mehrere gleichartige oder verschiedene Atome oder Gruppen ausgewählt aus Halogenatomen, geradkettigen oder verzweigten ($C_1$-$C_6$)-Alkylgruppen, Hydroxygruppen, geradkettigen oder verzweigten ($C_1$-$C_6$)-Alkoxygruppen, geradkettigen oder verzweigte ($C_1$-$C_6$)-Polyhalogenalkylgruppen oder Aminogruppen (die gegebenenfalls durch eine oder mehrere geradkettige oder verzweigte ($C_1$-$C_6$)-Alkylgruppen substituiert sind) substituiert sein kann,
und man unter einem Stickstoff-haltigen Heterocyclus eine monocyclische gesättigte Gruppe mit 5 bis 7 Kettengliedern versteht, die eins, zwei oder drei Heteroatome enthält, wobei eines dieser Heteroatome Stickstoff ist und das oder die gegebenenfalls zusätzlich vorhandenen Heteroatome aus Sauerstoffatomen, Stickstoffatomen oder Schwefelatomen ausgewählt sind.

**2.** Verbindung der Formel (I) nach Anspruch 1, worin $R_0$ ein Wasserstoffatom bedeutet.

**3.** Verbindung der Formel (I) nach irgendeinem der Ansprüche 1 oder 2, worin $R_1$ und $R_2$, die gleichartig oder verschieden sind, jeweils:

- ein Wasserstoffatom.
- ein Halogenatom,
- eine geradkettige oder verzweigte ($C_1$-$C_6$)-Alkylgruppe,
- eine geradkettige oder verzweigte ($C_1$-$C_6$)-Alkoxygruppe,
- eine Hydroxygruppe,
- eine geradkettige oder verzweigte ($C_1$-$C_6$)-Polyhalogenalkylgruppe,
- eine Nitrogruppe,
- eine Aminogruppe, die gegebenenfalls durch eine oder zwei geradkettige oder verzweigte ($C_1$-$C_6$)-Alkylgruppen substituiert ist, oder
- eine Gruppe der Formel

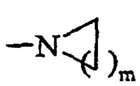,

in der m eine ganze Zahl mit Werten von $1 \leq m \leq 4$ darstellt.
bedeuten.

**4.** Verbindung der Formel (I) nach irgendeinem der Ansprüche 1 oder 2, worin $R_1$ und $R_2$ gemeinsam mit den sie tragenden Kohlenstoffatomen eine mono- oder bicyclische, aromatische oder nichtaromatische Gruppe mit 5 bis 12 Kettengliedern bilden, die gegebenenfalls 1 oder 2 Heteroatome ausgewählt aus O, S und N enthält.

**5.** Verbindung der Formel (I) nach Anspruch 4, worin $R_1$ und $R_2$ gemeinsam mit dem sie tragenden Kohlenstoffatom eine Gruppe der Formel $G_3$ oder $G_4$ bilden:

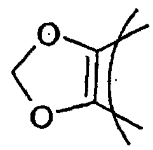

$G_3$

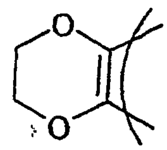

$G_4$

**6.** Verbindung der Formel (I) nach Anspruch 4, worin $R_1$ und $R_2$ gemeinsam mit den sie tragenden Kohlenstoffatomen eine Phenylengruppe bilden.

**7.** Verbindung der Formel (I) nach irgendeinem der Ansprüche 1 bis 6, worin X ein Sauerstoffatom darstellt.

**8.** Verbindung der Formel (I) nach irgendeinem der Ansprüche 1 bis 6, worin X ein Schwefelatom darstellt.

**9.** Verbindung der Formel (I) nach irgendeinem der Ansprüche 1 bis 6, worin X eine Gruppe $-CH_2-$ oder $-CH_2-CH_2-$ darstellt.

**10.** Verbindung der Formel (I) nach irgendeinem der Ansprüche 1 bis 9, worin Ar eine Arylgruppe darstellt.

**11.** Verbindung der Formel (I) nach irgendeinem der Ansprüche 1 bis 9, worin Ar eine Heteroarylgruppe darstellt.

**12.** Verbindung der Formel (I) nach Anspruch 10, worin Ar eine Phenylgruppe darstellt.

**13.** Verbindung der Formel (I) nach Anspruch 1, nämlich (±)-4-Methyl-6,7-methylendioxy-9-(3,4,5-trimethoxyphenyl)-4,9-dihydrofuro[3,4-b]chinolin-1(3*H*)-on, sowie dessen optische Isomere, dessen Hydrate und dessen Solvate.

**14.** Verbindung der Formel (I) nach Anspruch 1, nämlich (±)-6,7-Methylendioxy-9-(3,4,5-trimethoxyphenyl)-4,9-dihydrofuro[3,4-b]chinolin-1(3*H*)-on, dessen optische Isomere, dessen Hydrate, dessen Solvate sowie dessen Additionssalze mit einer pharmazeutisch annehmbaren Säure.

**15.** Verbindung der Formel (I) nach Anspruch 1, nämlich (±)-6-Methoxy-9-(3,4,5-trimethoxyphenyl)-4,9-dihydrofuro [3,4-b]chinolin-1(3*H*)-on, dessen optische Isomere, dessen Hydrate, dessen Solvate sowie dessen Additionssalze mit einer pharmazeutisch annehmbaren Säure.

**16.** Verbindung der Formel (I) nach Anspruch 1, nämlich (±)-7-(3,4,5-Trimethoxyphenyl)-7,11-dihydrobenzo[h]furo [3,4-b]chinolin-8(10*H*)-on, dessen optische Isomere, dessen Hydrate, dessen Solvate sowie dessen Additionssalze mit einer pharmazeutisch annehmbaren Säure.

**17.** Verbindung der Formel (I) nach Anspruch 1, nämlich (±)-6,7-Methylendioxy-9-(3,4,5-trimethoxyphenyl)-2,3,4,9-tetrahydrocyclopenta[b]chinolin-1-on, dessen optische Isomere, dessen Hydrate, dessen Solvate sowie dessen Additionssalze mit einer pharmazeutisch annehmbaren Säure.

**18.** Verbindung der Formel (I) nach Anspruch 1, nämlich (±)-6,7-Ethylendioxy-9-(3,4,5-trimethoxyphenyl)-4,9-dihydrofuro[3,4-b]chinolin-1(3H)-on, dessen optische Isomere, dessen Hydrate, dessen Solvate sowie dessen Additionssalze mit einer pharmazeutisch annehmbaren Säure.

**19.** Verbindung der Formel (I) nach Anspruch 1, nämlich (±)-7-(3,4,5-Trimethoxyphenyl)-7,9,10,11-tetrahydro-8*H*-benzo[h]cyclopenta[b]chinolin-8-on, dessen optische Isomere, dessen Hydrate, dessen Solvate sowie dessen Additionssalze mit einer pharmazeutisch annehmbaren Säure.

**20.** Verfahren zur Herstellung der Verbindungen der Formel (I) nach Anspruch 1, **dadurch gekennzeichnet, daß** man eine Verbindung der Formel (II):

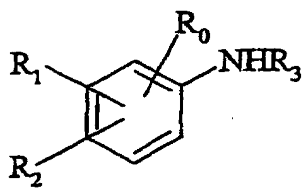

(II)

in der $R_0$, $R_1$, $R_2$ und $R_3$ die bezüglich der Formel (I) angegebenen Bedeutungen besitzen, mit einer Verbindung der Formel (III):

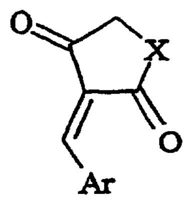

(III)

in der X und Ar die bezüglich der Formel (I) angegebenen Bedeutungen besitzen, umsetzt zur Bildung der Verbindung der Formel (I), welche man gegebenenfalls mit Hilfe einer klassischen Reinigungsmethode reinigt, welche man gewünschtenfalls mit Hilfe einer klassischen Trennmethode in ihre optischen Isomeren auftrennt und welche man gewünschtenfalls in ihre Additionssalze mit einer pharmazeutisch annehmbaren Säure überführt.

**21.** Verfahren zur Herstellung der Verbindungen der Formel (I) nach Anspruch 1, **dadurch gekennzeichnet, daß** man in einem Eintopfverfahren eine Verbindung der Formel (II):

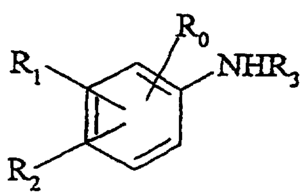

(II)

in dei $R_0$, $R_1$, $R_2$ und $R_3$ die bezüglich der Formel (I) angegebenen Bedeutungen besitzen, mit einer Verbindung der Formel (VIII):

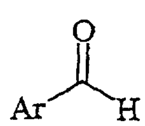

(VIII)

in der Ar die bezüglich der Formel (I) angegebenen Bedeutungen besitzt, und einer Verbindung der Formel (IX):

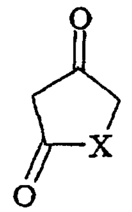

(IX)

in der X bezüglich der Formel (I) angegebenen Bedeutungen besitzt,
umsetzt zur Bildung der Verbindung der Formel (I),
welche man gegebenenfalls mit Hilfe einer klassischen Reinigungsmethode reinigt, welche man gewünschtenfalls mit Hilfe einer klassischen Trennmethode inihre optischen Isomeren trennt und welche man gewünschtenfalls in ihre Additionssalze mit einer pharmazeutisch annehmbaren Säure überführt.

**22.** Pharmazeutische Zubereitung enthaltend als Wirkstoff eine Verbindung nach einem der Ansprüche 1 bis 19, allein oder in Kombination mit einem oder mehreren inerten, nichttoxischen und pharmazeutisch annehmbaren Trägermaterialien.

**23.** Pharmazeutische Zubereitung nach Anspruch 22 zur Herstellung von Antikrebs-Arzneimitteln.